# EUROPEAN PATENT APPLICATION

(11) **EP 1 825 866 A1**
(43) Date of publication of application: **29.08.2007**
(21) Application number: 05811622.9
(22) Date of filing: 02.12.2005
(51) Int. Cl.: A61K 47/12, A61K 47/04, A61K 47/38, A61K 9/20, A61K 31/4178

(54) **SOLID PREPARATION**

(30) Priority: 03.12.2004 JP 2004350972
(71) Applicant: Takeda Pharmaceutical Company Limited, Osaka-shi, Osaka 541-0045 (JP)
(72) Inventor: UCHIYAMA, Yoshihiro TAKEDA PHARM. COMPANY LIMITED, Osaka-shi, Osaka 5328686 (JP); YOSHINARI, Tomohiro TAKEDA PHARM. COMPANY LIMITED, Osaka-shi, Osaka 5328686 (JP); FUKUTA, Makoto TAKEDA PHARM. COMPANY LIMITED, Osaka-shi, Osaka 5328686 (JP)
(74) Representative: Rickard, Timothy Mark Adrian
(86) International application number: PCT/JP2005/022187
(87) International publication number: WO 2006/059716

(57) **Abstract**

A medical drug, in particular, solid preparations containing a medicinal ingredient with high tendency toward gelation, **characterized by** simultaneously containing a surface modifier and an acid or base. This characteristic realizes improvement to the disintegration easiness, production efficiency and stability of the solid preparations containing the above medicinal ingredient.

## Description

### Technical Field

The present invention relates to a solid preparation. More specifically, it relates to a solid preparation comprising a medicinal ingredient with tendency toward gelation.

### Background Art

Some medicinal ingredients have tendency toward gelation and, in case of solid preparations containing such types of medicinal ingredients, absorption rates of the ingredients in the body are significantly reduced. That is, when a solid preparation is administered orally, usually, it is disintegrated in the gastrointestinal tract, allowing dissolution of the medicinal ingredient and its absorption into the body. However, in case of oral administration of a solid preparation containing a medicinal ingredient with tendency toward gelation, the disintegration of the solid preparation is interfered with gelation of the medicinal ingredient, which causes significant reduction of an absorption rate of the medicinal ingredient.

The present inventors studied intensively so as to solve the above problem. As a result, the present inventors found that gelation could be prevented by blending an acid or base in a solid preparation containing a medicinal ingredient with tendency toward gelation.

However, it became apparent that when an acid or base was blended in a solid preparation, the disintegration properties of the preparation were deteriorated because of the influence of the acid or base, and that problems were caused in a compression process of tablets (specifically, adhesion of tablets to punch), resulting in a loss of workability of the preparation. In addition, when an acid or base was blended in a solid preparation, there were problems of deliquescence or hygroscopicity, and a loss of preparation stability.

Thus, the present inventors have further studied intensively and, as a result, have found that these problems can be solved by further blending a surface modifier in addition to an acid or base.

### Disclosure of the Invention

### Problem to be Solved by the Invention

An object of the present invention is to improve the disintegration properties of a solid preparation containing a medicinal ingredient with tendency toward gelation.

Another object of the present invention is to improve the dissolution properties of a solid preparation containing a medicinal ingredient with tendency toward gelation.

Yet another object of the present invention is to improve the workability of the above solid preparation having improved disintegration properties.

Still another object of the present invention is to improve stability in the above solid preparation having improved disintegration properties.

### Means for Solving the Problem

The present invention provides:
1. A solid preparation comprising a medicinal ingredient having tendency toward gelation, a surface modifier, and an acid or base;
2. The solid preparation according to the above 1, wherein the medicinal ingredient having tendency toward gelation is a salt of a compound that is extremely poorly soluble in the state of its free form;
3. The solid preparation according to the above 1, wherein the medicinal ingredient having tendency toward gelation is a salt of an amphoteric or basic compound, and the acid or base is an acid;
4. The solid preparation according to the above 2, wherein the salt is that formed with hydrochloric acid, hydrobromic acid, nitric acid, sulfuric acid, phosphoric acid, formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, arginine, lysine, or ornithine;
5. The solid preparation according to the above 1, wherein the amphoteric or basic compound, or salt thereof, is a compound represented by the formula (I): 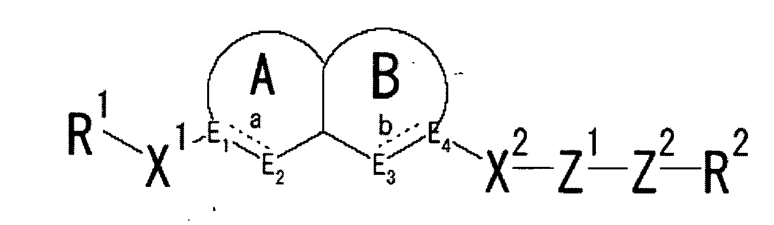 wherein R¹ denotes an optionally substituted 5- to 6-membered ring,
   X¹ denotes a bond or a divalent group wherein the number of atoms constituting the straight-chain moiety is 1 to 4, ring A denotes an optionally substituted 5- or 6-membered ring and ring B denotes an optionally substituted 8- to 10-membered ring,
   E₁ and E₄ each denote an optionally substituted carbon atom or an optionally substituted nitrogen atom,
   E₂ and E₃ each denote an optionally substituted carbon atom, an optionally substituted nitrogen atom, an optionally oxidized sulfur atom or an oxygen atom,
   a and b each denote a single bond or a double bond,
   X² denotes a divalent group wherein the number of atoms constituting the straight-chain moiety is 1 to 4,
   Z¹ denotes a bond or a divalent cyclic group,
   Z² denotes a bond or a divalent group,
   R² denotes (1) an optionally substituted amino group wherein the nitrogen atom may be converted into a quaternary ammonium or oxide, (2) an optionally substituted nitrogen-containing heterocyclic group which may contain a sulfur atom or an oxygen atom as a ring-constituent atom and wherein the nitrogen atom may be converted into a quaternary ammonium or oxide, (3) a group represented by the formula: 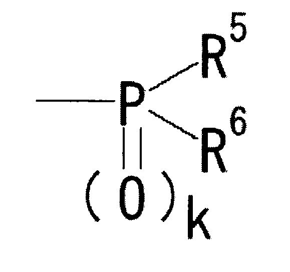 wherein k denotes 0 or 1, and when k is 0, the phosphorus atom can form a phosphonium salt, R⁵ and R⁶ each denote an optionally substituted hydrocarbon group, an optionally substituted hydroxyl group, or an optionally substituted amino group, or R⁵ and R⁶ can bond each other to form a cyclic group together with the adjacent phosphorus atom, (4) an optionally substituted amidino group, or (5) an optionally substituted guanidino group, or a salt thereof;
6. The solid preparation according to the above 1, wherein the acid or base is a solid;
7. The solid preparation according to the above 1, wherein the acid is a carboxylic acid, sulfonic acid, an acidic polysaccharide, or an acidic amino acid;
8. The solid preparation according to the above 1, wherein the acid is a carboxylic acid;
9. The solid preparation according to the above 8, wherein the carboxylic acid is fumaric acid, adipic acid, malic acid, acetic acid, tartaric acid, succinic acid or citric acid;
10. The solid preparation according to the above 9, wherein the carboxylic acid is citric acid;
11. The solid preparation according to the above 1, which comprises 0.1 to 20 parts by weight of the acid or base based on 1 part by weight of the medicinal ingredient with tendency toward gelation;
12. The solid preparation according to the above 1, which comprises 0.05 to 20 parts by weight of the surface modifier based on 1 part by weight of the medicinal ingredient with tendency toward gelation;
13. The solid preparation according to the above 1, which is a tablet;
14. The solid preparation according to the above 1, which is a coated preparation;
15. The solid preparation according to the above 1, wherein the content of the acid or base is 2 to 85% (w/w) based on the total preparation;
16. The solid preparation according to the above 1, wherein the content of the acid or base is 2 to 85% (w/w) based on a plain tablet;
17. The solid preparation according to the above 1, which further comprises talc and/or magnesium stearate;
18. A process for producing a solid preparation which comprises a medicinal ingredient with tendency toward gelation a surface modifier, and an acid or base, wherein at least the acid or base is mixed in advance with colloidal silicon dioxide;
19. The process according to the above 18, wherein the average particle diameter of the surface modifier is as large as 1/5 or less of that of the medicinal ingredient with tendency toward gelation in the form of a powder;
20. The process according to the above 18, wherein the average particle diameter of the surface modifier is as large as 1/5 or less of that of the acid or base in the form of a powder;
21. The process according to the above 18, wherein the average particle diameter of the surface modifier is about 5 nm to 5 µm; and the like

### Effect of the Invention

According to the present invention, it is possible to improve the disintegration properties of a solid preparation comprising a medicinal ingredient with tendency of gelation.
According to the present invention, it is possible to improve the dissolution properties of a solid preparation containing a medicinal ingredient with tendency toward gelation.
According to the present invention, it is possible to improve the workability of the above solid preparation having improved disintegration properties.
According to the present invention, it is possible to improve the stability of the above solid preparation having improved disintegration properties.

### Brief Description of Drawings

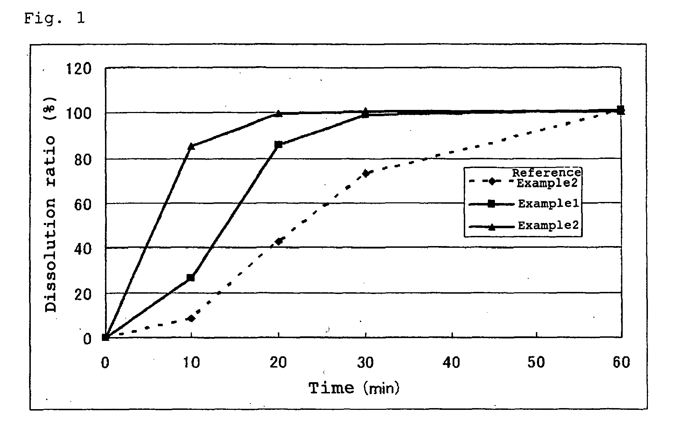

Fig. 1 is a graph showing the dissolution ratio of compound A.

### Best Mode for Carrying Out the Invention

The term "medicinal ingredient with tendency toward gelation" used herein refers to a medicinal ingredient has tendency toward gelation in the presence of water, that is, when it is exposed to, for example, drinking water or body fluid such as saliva, gastric juices, and the like.

As the "medicinal ingredient with tendency toward gelation" suitable for use in the present invention, there are salts of compounds that are extremely poorly soluble when in their free form. The term "extremely poorly soluble" refers to such properties that the solubility is 10⁻³ mg/mL or less, preferably 10⁻⁴ mg/mL or less, and more preferably 10⁻⁵ mg/mL or less under any conditions to which the compound is exposed in the body. In order to manifests the tendency toward gelation, a salt of a compound must exhibit higher solubility than the free form of the compound. A compound tends to have higher tendency toward gelation as the difference in solubility levels between the two forms increases (e.g., 1000-fold, preferably 10,000-fold, and more preferably 100,000-fold).

The solubility of the medicinal ingredient can be obtained as follows. After suspending 10 mg of the medicinal ingredient into 10 mL of purified water under the condition of 20°C, the obtained suspension is filtrated. The amount of the dissolved medicinal ingredient in purified water is measured by high performance liquid chromatography.

Examples of the salt include salts with inorganic bases (e.g., alkali metal salt such as sodium salt, potassium salt, etc.; alkaline earth metal salt such as calcium salt, magnesium salt, etc.; as well as an aluminum salt; and an ammonium salt), salts with organic bases (e.g., trimethylamine, triethylamine, pyridine, picoline, ethanolamine, diethanolamine, triethanolamine, dicyclohexylamine, and N,N'-dibenzylethylenediamine), salts with inorganic acids (e.g., hydrochloric acid, hydrobromic acid, sulfuric acid, and phosphoric acid), salts with organic acids (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid), and salts with basic amino acids (e.g., arginine, lysine, and ornithine) or acidic amino acids (aspartic acid and glutamic acid), and the like.

Examples of the "medicinal ingredient with tendency toward gelation" include a salt of a known compound disclosed WO 03/055525 A, and prepared by the process disclosed therein, which is a compound represented by the formula (I): 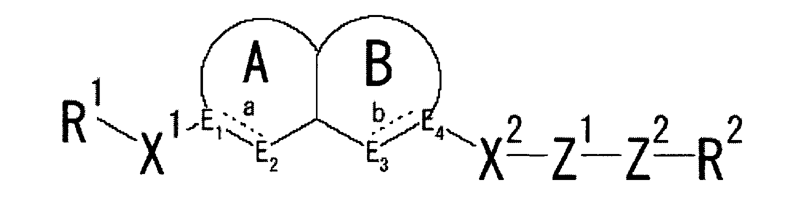 wherein R¹ denotes an optionally substituted 5- to 6-membered ring,
X¹ denotes a bond or a divalent group wherein the number of atoms constituting the straight-chain moiety is 1 to 4, ring A denotes an optionally substituted 5- or 6-membered ring and ring B denotes an optionally substituted 8- to 10-membered ring,
E₁ and E₄ each denote an optionally substituted carbon atom or an optionally substituted nitrogen atom,
E₂ and E₃ each denote an optionally substituted carbon atom, an optionally substituted nitrogen atom, an optionally oxidized sulfur atom or an oxygen atom,
a and b each denote a single bond or a double bond,
X² denotes a divalent group wherein the number of atoms constituting the straight-chain moiety is 1 to 4,
Z¹ denotes a bond or a divalent cyclic group,
Z² denotes a bond or a divalent group,
R² denotes (1) an optionally substituted amino group wherein the nitrogen atom may be converted into a quaternary ammonium or oxide, (2) an optionally substituted nitrogen-containing heterocyclic group which may contain a sulfur atom or an oxygen atom as a ring-constituent atom and wherein the nitrogen atom may be converted into a quaternary ammonium or oxide, (3) a group represented by the formula: 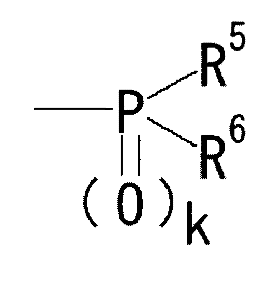 wherein k denotes 0 or 1, and when k is 0, the phosphorus atom can form a phosphonium salt, R⁵ and R⁶ each denote an optionally substituted hydrocarbon group, an optionally substituted hydroxyl group, or an optionally substituted amino group, or R⁵ and R⁶ can bond each other to form a cyclic group together with the adjacent phosphorus atom, (4) an optionally substituted amidino group, or (5) an optionally substituted guanidino group.

Hereinafter, examples of halogen include fluorine, chlorine, bromine, and iodine.

Examples of the "5- to 6-membered ring" in the "optionally substituted 5- to 6-membered ring" represented by R¹ in the above formula (I) include groups formed by the removal of one hydrogen atom from 6-membered aromatic hydrocarbons such as benzene, etc.; 5- to 6-membered aliphatic hydrocarbons such as cyclopentane, cyclohexane, cyclopentane, cyclohexene, cyclopentadiene, cyclohexadiene, etc.; 5- to 6-membered aromatic heterocyclic rings having 1 to 4 hetero atoms of 1 to 2 types selected from a nitrogen atom, a sulfur atom and an oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; 5- to 6-membered non-aromatic heterocyclic rings having 1 to 4 hetero atoms of 1 to 2 types selected from a nitrogen atom, a sulfur atom and an oxygen atom such as tetrahydrofuran, tetrahydrothiophene, dithiolane, oxathiolane, pyridine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.; and the like. Among them, as the "5- to 6-membered ring", the preferred are benzene, furan, thiophene, pyridine, cyclopentane, cyclohexane, pyrrolidone, piperidine, piperazine, morpholine, thiomorpholine, tetrahydrofuran, (preferably 6-membered rings), and the like, with benzene being particularly preferred.

Examples of the "substituent" which the "5- to 6-membered ring" of the "optionally substituted 5- to 6-membered ring" represented by R¹ may have include halogen, nitro, cyano, optionally substituted alkyl, optionally substituted cycloalkyl, an optionally substituted hydroxyl group, an optionally substituted thiol group (wherein the sulfur atom may be oxidized to form an optionally substituted sulfinyl group or an optionally substituted sulfonyl group), an optionally substituted amino group, an optionally substituted acyl group, an optionally esterified carboxyl group, an optionally substituted aromatic group, and the like.

Examples of the halogen as the substituent in R¹ include that described above, with fluorine and chlorine being particularly preferred.

Examples of the alkyl of the optionally substituted alkyl as the substituent in R¹ are linear or branched C₁₋₁₀ alkyls, for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., with lower (C₁₋₆) alkyl being preferred. Examples of the substituent of the optionally substituted alkyl include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkoxy (e.g., methoxy, ethoxy, propoxy, butoxy, trifluoromethoxy, trifluoromethoxy, etc.), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), or the like, and the number of substituents is preferably 1 to 3.

Examples of the cycloalkyl of the optionally substituted cycloalkyl as a substituent in R¹ include C₃₋₇ cycloalkyl such as cyclopropyl, cyclobutyl, cyclopentyl, cyclohexyl, cycloheptyl, and the like. Examples of the substituent of the optionally substituted cycloalkyl include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkoxy (e.g., methoxy, ethoxy, propoxy, butoxy, trifluoromethoxy, trifluoroethoxy, etc.), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like, and the number of substituents is preferably 1 to 3.

Examples of the substituents of the optionally substituted hydroxyl group as the substituent in R¹ include:
(1) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl;
(2) optionally substituted cycloalkyl that may have a heteroatom (e.g., C₃₋₇ cycloalkyl such as cyclopropyl, cyclobutyl, cyclopentyl, cyclohexyl, cycloheptyl, etc., a saturated 5- to 6-membered heterocyclic group having 1 to 2 heteroatoms such as tetrahydrofuranyl, tetrahydrothienyl, pyrrolidinyl, pyrazolidinyl, piperidyl, piperazinyl, morpholinyl, thiomorpholinyl, tetrahydropyranyl, tetrahydrothiopyranyl, (preferably tetrahydropyranyl, etc.) or the like);
(3) optionally substituted alkenyl (e.g., C₂₋₁₀ alkenyl such as allyl, crotyl, 2-pentenyl, 3-hexenyl, etc., preferably lower (C₂₋₆) alkenyl);
(4) optionally substituted cycloalkenyl (e.g., C₃₋₇ cycloalkenyl such as 2-cyclopentenyl, 2-cyclohexenyl, 2-cyclopentenylmethyl, 2-cyclohexenylmethyl, etc.);
(5) optionally substituted aralkyl (e.g., phenyl-C₁₋₄ alkyl (e.g., benzyl, phenethyl, etc.), etc.);
(6) formyl or optionally substituted acyl (e.g., C₂₋₄ alkanoyl (e.g., acetyl, propionyl, butyryl, isobutyryl, etc.), C₁₋₄ alkylsulfonyl (e.g., methanesulfonyl, ethanesulfonyl, etc.), or the like); or
(7) optionally substituted aryl (e.g., phenyl, naphthyl, etc.), and the like.

Examples of the substituent which (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, and (7) optionally substituted aryl may have include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkyl (e.g., trifluoromethyl, methyl, ethyl, etc.), optionally halogenated C₁₋₆ alkoxy (e.g., methoxy, ethoxy, propoxy, butoxy, trifluoromethoxy, trifluoromethoxy, etc., preferably optionally halogenated C₁₋₄ alkoxy), formyl, C₂₋₄ alkanoyl (e.g., acetyl, propionyl, etc.), C₁₋₄ alkylsulfonyl (e.g., methanesulfonyl, ethanesulfonyl, etc.), an optionally substituted 5- to 6-membered aromatic heterocyclic group {e.g., 5- to 6-membered aromatic heterocyclic ring having 1 to 4 heteroatoms of 1 to 2 types selected from a nitrogen atom, a sulfur atom, and an oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc., and examples of the substituent of the aforementioned heterocyclic ring include halogen, nitro, cyano, hydroxyl, thiol, amino, carboxyl, optionally halogenated C₁₋₄ alkoxy (e.g., trifluoromethyl, methyl, ethyl, etc.), optionally halogenated C₁₋₄ alkoxy (e.g., methoxy, ethoxy, propoxy, butoxy, trifluoromethoxy, trifluoroethoxy, etc.), formyl, C₂₋₄ alkanoyl (e.g., acetyl, propionyl, etc.), C₁₋₄ alkylsulfonyl (e.g. a methanesulfonyl, ethanesulfonyl, etc.) or the like, and the number of substituents is preferably 1 to 3}, or the like. The number of substituents is preferably 1 to 3.

Examples of the substituent of the optionally substituted thiol group as the substituent in R¹ include the same substituents as those exemplified with respect to the above "substituent of the optionally substituted hydroxyl group as the substituent in R¹". Among them, preferred are:
(1) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) optionally substituted cycloalkyl (e.g., C₃₋₇ cycloalkyl such as cyclopropyl, cyclobutyl, cyclopentyl, cyclohexyl, cycloheptyl, etc.);
(3) optionally substituted aralkyl (e.g., phenyl-C₁₋₄ alkyl (e.g., benzyl or phenethyl), etc.); and
(4) optionally substituted aryl (e.g., phenyl, naphthyl, etc.), and the like. Examples of the substituent which the (1) optionally substituted alkyl, (2) optionally substituted cycloalkyl, (3) optionally substituted aralkyl and (4) optionally substituted aryl may have include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl or di-C₁₋₄ alkyl-carbamoyl, optionally halogenated C₁₋₄ alkoxy (e.g., methoxy, ethoxy, propoxy, butoxy, trifluoromethoxy, trifluoroethoxy, etc.), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like, and the number of substituents is preferably 1 to 3.

Examples of the substituent of the optionally substituted amino group as the substituent in R¹ include an amino group having the same one to two substituents as the aforementioned "substituent of the optionally substituted hydroxyl group as the substituent in R¹", and the like. Among them, preferred are
(1) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, or the like);
(2) optionally substituted cycloalkyl (e.g., C₃₋₇ cycloalkyl such as cyclopropyl, cyclobutyl, cyclopentyl, cyclohexyl, cycloheptyl, etc.);
(3) optionally substituted alkenyl (e.g., C₂₋₁₀ alkenyl such as allyl, crotyl, 2-pentenyl, 3-hexenyl, etc., preferably lower (C₂₋₆) alkenyl or the like);
(4) optionally substituted cycloalkenyl (e.g., C₃₋₇ cycloalkenyl such as 2-cyclopentenyl, 2-cyclohexenyl, 2-cyclopentenylmethyl, 2-cyclohexenylmethyl, etc.);
(5) formyl or optionally substituted acyl (e.g., C₂₋₄ alkanoyl (e.g., acetyl, propionyl, butyryl, isobutyryl, etc.), C₁₋₄ alkylsulfonyl (e.g., methanesulfonyl, ethanesulfonyl, etc.), and the like); and
(6) optionally substituted aryl (e.g., phenyl, naphthyl, etc.), and the like.

Examples of the substituent which the (1) optionally substituted alkyl, (2) optionally substituted cycloalkyl, (3) optionally substituted alkenyl, (4) optionally substituted cycloalkenyl, (5) optionally substituted acyl, and (6) optionally substituted aryl may have include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkoxy (e.g., methoxy, ethoxy, propoxy, butoxy, trifluoromethoxy, trifluoroethoxy, etc.), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like, and the number of substituents is preferably 1 to 3.

Further, in the optionally substituted amino group as the substituent in R¹, two substituents of the amino group may bond to each other to form a cyclic amino group (e.g., a cyclic amino group formed by removing one hydrogen atom from the ring-forming nitrogen atom of a 5- to 6-membered ring such as tetrahydropyrrole, piperazine, piperidine, morpholine, thiomorpholine, pyrrole, imidazole, etc., which bonds to the 5- or 6-membered ring through the nitrogen atom, and the like). This cyclic amino group may have substituent(s), and examples of the substituent include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkoxy (e.g., methoxy, ethoxy, propoxy, butoxy, trifluoromethoxy, trifluoroethoxy, etc.), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like, and the number of substituents is preferably 1 to 3.

Examples of the optionally substituted acyl as the substituent in R¹ include a carbonyl group or a sulfonyl group which is bonded to:
(1) hydrogen;
(2) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, or the like);
(3) optionally substituted cycloalkyl (e.g., C₃₋₇ cycloalkyl such as cyclopropyl, cyclobutyl, cyclopentyl, cyclohexyl, cycloheptyl, etc.);
(4) optionally substituted alkenyl (e.g., C₂₋₁₀ alkenyl such as allyl, crotyl, 2-pentenyl, 3-hexenyl, etc., preferably lower (C₂₋₆) alkenyl, or the like);
(5) optionally substituted cycloalkenyl (e.g., C₃₋₇ cycloalkyl such as 2-cyclopentenyl, 2-cyclohexyl, 2-cyclopentenylethyl, 2-cyclohexenylmethyl, etc.);
(6) an optionally substituted 5- to 6-membered monocyclic aromatic group (e.g., phenyl, pyridyl, etc.), and the like (e.g., formyl, acetyl, propionyl, butyryl, isobutyryl, valeryl, isovaleryl, pivaloyl, hexanoyl, heptanoyl, octanoyl, cyclobutane carbonyl, cyclopentane carbonyl, cyclohexane carbonyl, cycloheptane carbonyl, crotonyl, 2-cyclohexene carbonyl, benzoyl, nicotinoyl, methanesulfonyl, ethanesulfonyl, etc.). Examples of the substituent which the above (2) optionally substituted alkyl, (3) optionally substituted cycloalkyl, (4) optionally substituted alkenyl, (5) optionally substituted cycloalkenyl, or (6) an optionally substituted 5- to 6-membered monocyclic aromatic group may have include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkoxy (e.g., methoxy, ethoxy, propoxy, butoxy, trifluoromethoxy, trifluoroethoxy, etc.), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like, and the number of substituents is preferably 1 to 3.

Examples of the optionally esterified carboxyl group as the substituent in R¹ include a carbonyloxy group which is bonded to
(1) hydrogen;
(2) optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, or the like);
(3) optionally substituted cycloalkyl (e.g., C₃₋₇ cycloalkyl such as cyclopropyl, cyclobutyl, cyclopentyl, cyclohexyl, cycloheptyl, etc.);
(4) optionally substituted alkenyl (e.g., C₂₋₁₀ alkenyl such as allyl, crotyl, 2-pentenyl, 3-hexenyl, etc., preferably lower (C₂₋₆) alkenyl, or the like);
(5) optionally substituted cycloalkenyl (e.g., C₃₋₇ cycloalkenyl such as 2-cyclopentenyl, 2-cyclohexyl, 2-cyclopentenylethyl, 2-cyclohexenylmethyl, etc.); or
(6) optionally substituted aryl (e.g., phenyl, naphthyl, etc.), and the like, and preferably a carboxyl, lower (C₁₋₆) alkoxycarbonyl, aryloxycarbonyl (e.g., methoxycarbonyl, ethoxycarbonyl, propoxycarbonyl, phenoxycarbonyl, naphthoxycarbonyl, etc.), and the like. Examples of the substituent which (2) optionally substituted alkyl, (3) optionally substituted cycloalkyl, (4) optionally substituted alkenyl, (5) optionally substituted cycloalkenyl or (6) optionally substituted aryl may have include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkoxy (e.g., methoxy, ethoxy, propoxy, butoxy, trifluoromethoxy, trifluoroethoxy, etc.), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like. The number of substituents is preferably 1 to 3.

Examples of the aromatic group in the optionally substituted aromatic group as the substituent in R¹ include a 5- to 6-membered homocyclic or heterocyclic aromatic group such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl, triazolyl, etc.; a condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; and the like. Examples of the substituent of the aromatic group include halogen, nitro, cyano, hydroxyl, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkyl (e.g., trifluoromethyl, methyl, ethyl, etc.), optionally halogenated C₁₋₄ alkoxy (e.g., methoxy, ethoxy, propoxy, butoxy, trifluoromethoxy, trifluoroethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like. The number of substituents is preferably 1 to 3.

The number of the substituents of R¹ may be 1 to 4 (preferably 1 to 2) and the substituents may be the same or different at any positions of the ring. In addition, when the "5- to 6-membered ring" of the "optionally substituted 5- to 6-membered ring" represented by R¹ has two or more substituents, two of these substituents may bond to each other to form lower (C₁₋₆) alkylene (e.g., trimethylene, tetramethylene, etc.), lower (C₁₋₆) alkyleneoxy (e.g., -CH₂-O-CH₂-, -O-CH₂-CH₂-, -O-CH₂-CH₂-CH₂-, -O-CH₂-CH₂-CH₂-CH₂-, -O-C(CH₃)(CH₃)-CH₂-CH₂-, etc.), lower (C₁₋₆) alkylenethio (e.g., -CH₂-S-CH₂-, -S-CH₂-CH₂-, -S-CH₂-CH₂-CH₂-, -S-CH₂-CH₂-CH₂-CH₂-, -S- (C (CH₃) (CH₃)-CH₂-CH₂-, etc.), lower (C₁₋₆) alkylenedioxy (e.g., -O-CH₂-O-, -O-CH₂-CH₂-O-, -O-CH₂-CH₂-CH₂-O-, etc.), lower (C₁₋₆) alkylenedithio (e.g., -S-CH₂-S-, -S-CH₂-CH₂-S-, -S-CH₂-CH₂-CH₂-S-, etc.), oxy-lower (C₁₋₆) alkyleneamino (e.g., -O-CH₂-NH-, -O-CH₂-CH₂-NH, etc.), oxy-lower (C₁₋₆) alkylenethio (e.g., -O-CH₂-S-, -O-CH₂-CH₂-S-, etc.), lower (C₁₋₆) alkyleneamino (e.g., -NH-CH₂-CH₂-, -NH-CH₂-CH₂-CH₂-, etc.), lower (C₁₋₆) alkylenediamino (e.g., -NH-CH₂-NH-, -NH-CH₂-CH₂-NH, etc.), thia-lower (C₁₋₆) alkyleneamino (e.g., - S-CH₂-NH-, -S-CH₂-CH₂-NH, etc.), lower (C₂₋₆) alkenylene (e.g., -CH₂-CH=CH-, -CH₂-CH₂-CH=CH-, -CH₂-CH=CH-CH₂, etc.), lower (C₄₋₆) alkadienylene (e.g., -CH=CH-CH=CH-, etc.), and the like.

Further, the divalent group formed by the bonding of two substituents of R¹ may have 1 to 3 substituents of the same as the "substituents" which the "5- to 6-membered ring" of the "optionally substituted 5- to 6-membered ring" may have (e.g., a halogen atom, nitro, cyano, optionally substituted alkyl, optionally substituted cycloalkyl, optionally substituted hydroxyl, an optionally substituted thiol group (where the sulfur atom may be oxidized, and may form an optionally substituted sulfinyl group or optionally substituted sulfonyl group), an optionally substituted amino group, optionally substituted acyl, an optionally esterified or amidated carboxyl group, an optionally substituted aromatic group, or the like).

Inter alia, examples of the "substituent" which the "5- to 6-membered ring" of the "optionally substituted 5-to 6-membered ring" represented by R¹ may have include optionally halogenated (C₁₋₄) alkyl or optionally lower (C1-4) alkoxylated lower (C₁₋₄) alkyl (e.g., methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, etc.), optionally halogenated (C₁₋₄) alkyl or optionally lower (C1-4) alkoxylated lower (C₁₋₄) alkoxy (e.g., methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), halogen (e.g., fluorine, chlorine, etc.), nitro, cyano, amino optionally substituted with 1 to 2 lower (C₁₋₄) alkyl groups, amino which may be substituted with formyl or lower (C₂₋₄) alkanoyl (e.g., amino, methylamino, dimethylamino, formylamino, acetylamino, etc.), a 5- to 6-membered cyclic amino group (e.g., 1-pyrrolidinyl, 1-piperazinyl, 1-piperidinyl, 4-morpholino, 4-thiomorpholino, 1-imidazolyl, 4-tetrahydropyranyl, etc.), and the like.

Examples of the "divalent group wherein the number of atoms constituting the straight chain moiety is 1 to 4" represented by X¹ and X² include -(CH₂)_{a'}- (where a' denotes an integer of 1 to 4 (with an integer of 1 to 2 being preferred)), -(CH₂)_{b'}-X ³_ {where b' denotes integer of 0 to 3 (preferably 0 or 1), and X³ denotes an optionally substituted imino group (e.g., an imino group that may be substituted with lower (C₁₋₆) alkyl, lower (C₃₋₇) cycloalkyl, formyl, lower (C₂₋₇) alkanoyl, lower (C₁₋₆) alkoxy-carbonyl, etc.), a carbonyl group, an oxygen atom, or an optionally oxidized sulfur atom (e.g., -S(O)ₘ- (where m denotes an integer of 0 to 2)}, -CH=CH-, -C≡C-, -CO-NH-, -SO₂-NH-, and the like. These groups may bond to ring A or ring B by either of their left or right bond, but with respect to X¹, it is preferable for bonding with ring A to occur via the right-side bond, and with respect to X², it is preferable for bonding with ring B to occur via the left-side bond.

X¹ is preferably a bond, -(CH₂)_{b'}-O- (where b' denotes an integer of 0, 1, or 2 (preferably 0 or 1)), -C≡C-, or the like, with a bond being more preferred.

X² is preferably -(CH₂)_{a'}- (where a denotes an integer of 1 to 2), -(CH₂)_{b'}-X³- (where b' denotes 0 or 1 and X³ denotes an optionally substituted an imino group, a carbonyl group, an oxygen atom, or an optionally oxidized sulfur atom), -CH=CH, -CO-NH-, -SO₂-NH-, or the like, with -CO-NH- being more preferred.

The divalent group represented by X¹ and X² may have a substituent at any position (preferably on a carbon atom), and examples of the substituent include any substituent that can bond to the divalent chain that constitutes the straight chain moiety. Further examples include lower (C₁₋₆) alkyl (e.g., methyl, ethyl, propyl, isopropyl, butyl, isobutyl, sec-butyl, tert-butyl, pentyl, isopentyl, neopentyl, hexyl, etc.), lower (C₃₋₇) cycloalkyl (e.g., cyclopropyl, cyclobutyl, cyclopentyl, cyclohexyl, cycloheptyl, etc.), formyl, lower (C₂₋₇) alkanoyl (e.g., acetyl, propionyl, butyryl, etc.), an optionally esterified phosphono group, an optionally esterified carboxyl group, carboxyl group, oxo, and the like, and preferably lower (C₁₋₆) alkyl (preferably C₁₋₃ alkyl), hydroxyl, oxo, and the like.

Examples of the above optionally esterified phosphono group include -P (O) (OR⁷) (OR⁸) (wherein R⁷ and R⁸ each denote hydrogen, a C₁₋₆ alkyl group, or a C₃₋₇ cycloalkyl group, and R⁷ and R⁸ may bond to each other to form a 5- to 7-membered ring).

In the above formula, examples of the C₁₋₆ alkyl group represented by R⁷ and R⁸ include methyl, ethyl, propyl, isopropyl, butyl, isobutyl, sec-butyl, tert-butyl, pentyl, isopentyl, neopentyl, hexyl, and the like, and examples of the C₃₋₇ cycloalkyl include cyclopropyl, cyclobutyl, cyclopentyl, cyclohexyl, cycloheptyl, and the like, but chain-form lower (C₁₋₆) alkyl is preferred, and lower (C₁₋₃) alkyl is more preferred. R⁷ and R⁸ may be the same or different, but preferably, they are the same. When R⁷ and R⁸ bond to each other to form a 5- to 7-membered ring, R⁷ and R⁸ bond to each other to form a linear C₂₋₄ alkylene side chain represented by -(CH₂)₂-, -(CH₂)₃-, or -(CH₂)₄-. This side chain may have substituent(s), and examples of the substituent include a hydroxyl group, halogen, and the like.

Examples of the esterified carboxyl group of the optionally esterified carboxyl group include a group produced by the bonding of a carboxyl group and a C₁₋₆ alkyl group or a C₃₋₇ cycloalkyl group. Examples thereof include methoxycarbonyl, ethoxycarbonyl propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, and the like.

Examples of the "5- to 6-membered ring" of the "optionally substituted 5- to 6-membered ring" represented by A in the above formula (I) include 5- to 6-membered saturated or unsaturated alicyclic hydrocarbons such as C₅₋₆ cycloalkane (e.g., cyclopentane, cyclohexane, etc.), C₅₋₆ cycloalkene (e.g., 1-cyclopentene, 2-cyclopentene, 3-cyclopentene, 2-cyclohexene, 3-cyclohexene, etc.), C₅₋₆ cycloalkadiene (e.g., 2,4-cyclopentadiene, 2,4-cyclohexadiene, 2,5-cyclohexadiene, etc.), and the like; 6-membered aromatic hydrocarbon such as benzene, and the like; 5- to 6-membered aromatic heterocyclic rings containing 1 to 3 types (preferably 1 or 2 types) of at least 1 (preferably 1 to 4, and more preferably 1 or 2) heteroatoms selected from an oxygen atom, a sulfur atom, a nitrogen atom, and the like; saturated or unsaturated non-aromatic heterocyclic rings (aliphatic heterocyclic rings); and the like.

Examples of the "aromatic heterocyclic ring" used herein include a 5- to 6-membered aromatic monocyclic heterocyclic ring (e.g., furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1, 2, 4-thiadiazole, 1,3,4-thiadiazole, 1,2,3-triazole, 1,2,4-triazole, tetrazole, pyridine, pyridazine, pyrimidine, pyrazine, triazine, etc.), and examples of the "non-aromatic heterocyclic ring" include 5- to 6-membered saturated or unsaturated non-aromatic heterocyclic rings (aliphatic heterocyclic rings) such as pyrrolidone, tetrahydrofuran, thiolane, piperidine, tetrahydropyran, morpholine, thiomorpholine, piperazine, pyran, oxepine, thiepine, azepine, and the like, or 5- to 6-membered non-aromatic heterocyclic rings wherein part or all of the double bonds of the above aromatic monocyclic heterocyclic rings are saturated, and the like.

The "5- to 6-membered ring" of the "optionally substituted 5- to 6-membered ring" represented by A are preferably 5- to 6-membered aromatic rings, and more preferably benzene, furan, thiophene, pyrrole, pyridine (preferably 6-membered rings), and the like, with benzene being the most preferred.

Examples of the "substituent" which the "5- to 6-membered ring" of the "optionally substituted 5- to 6-membered ring" represented by A may have is the same as the "substituent" which the "5- to 6-membered ring" of the "optionally substituted 5- to 6-membered ring" may have. Further, the number of the substituents of A may be 1 to 4 (preferably 1 to 2) and they may be the same or different at any positions of the ring. In addition, they may be at any positions regardless of the positions represented by E₁ and E₂ as far as they can be present.

Examples of the lower alkyl group of the "optionally substituted lower alkyl group" represented by R³ above include C₁₋₆ alkyl such as methyl, ethyl, propyl, isopropyl, butyl, isobutyl, sec-butyl, tert-butyl, pentyl, isopentyl, neopentyl, hexyl, and the like.

Examples of the lower alkoxy group of the "optionally substituted lower alkoxy group" represented by R³ above include C₁₋₆ alkoxy such as methoxy, ethoxy, propoxy, butoxy, and the like.

Examples of the substituent which the "optionally substituted lower alkyl group" and the "optionally substituted lower alkoxy group" may have include halogen (e.g., fluorine, chlorine, bromine, iodine, etc.), a hydroxyl group, amino, mono(lower alkyl)amino, di(lower alkyl)amino, lower alkanoyl, and the like.

Examples of the lower alkyl in the mono(lower alkyl)amino and the di(lower alkyl)amino include the same group as the lower alkyl group of the "optionally substituted lower alkyl group" represented by R³ above.

Examples of the lower alkanoyl include C₂₋₆ alkanoyl such as acetyl, propionyl, butyryl, isobutyryl, and the like.

Examples of the "halogen atom" represented by R³ above include fluorine, chlorine, bromine, iodine, and the like.

Among these groups, an optionally substituted lower C₁₋₆ alkyl group or a halogen atom is preferred for R³, and an optionally substituted methyl group or a halogen atom is more preferred.

Examples of the "8- to 10-membered ring" of the "optionally substituted 8- to 10-membered ring" represented by B in the formula (I) above include a 8- to 10-membered ring represented by the formula: 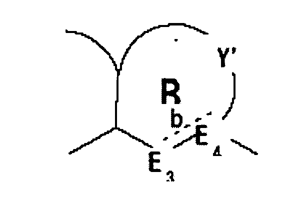 wherein Y' denotes a divalent group, and the other symbols are as defined as above, which may have substituent(s) at any possible position.

In the above formula, the divalent group represented by Y' denotes a divalent group whereby ring B forms an optionally substituted 8- to 10-membered ring, and examples thereof include:
(1) -Alkₐ₁-O-Alkₐ₂- (where Alkₐ₁ and Alkₐ₂ each denote a bond or a divalent linear hydrocarbon group with a carbon number of 1 to 5, and the sum of the carbon numbers of Alkₐ₁ and Alkₐ₂ is 5 or less),
(2) -Alk_{b1}-S(O)ₘ-Alk_{b2}- (where m denotes an integer of 0, 1, or 2; Alk_{b1} and Alk_{b2} each denote a bond or a divalent linear hydrocarbon group with a carbon number of 1 to 5; and the sum of the carbon numbers of Alk_{b1} and Alk_{b2} is 5 or less),
(3) -Alk_{d1} - (where Alk_{d1} denotes a divalent linear hydrocarbon group with a carbon number of 4-6),
(4) -Alkₑ₁-NH-Alkₑ₂- (Alkₑ₁ and Alkₑ₂ each denote a bond or a divalent linear hydrocarbon group with a carbon number of 1 to 5, and the sum of the carbon numbers of Alkₑ₁ and Alkₑ₂ is 5 or less), -Alkₑ₆-N=CH-Alkₑ₇-, -Alkₑ₇-CH=N-Alkₑ₆-, -Alkₑ₆-N=N-Alkₑ₇- (where Alkₑ₆ and Alkₑ₇ each denote a bond or a divalent linear hydrocarbon group with a carbon number of 1 to 4, and the sum of the carbon numbers of Alkₑ₆ and Alkₑ₇ is 4 or less), and the like.

Examples of these divalent linear hydrocarbon groups include divalent groups such as -CH₂-, -(CH₂)₂-, -(CH₂)₃-, - (CH₂)₄-, -(CH₂)₅-, -(CH₂)₆, -CH=, -CH=CH-, -CH=CH-CH₂-, - CH₂CH=CH-, -CH=CH-CH=CH-, =CH-CH=CH-, -CH₂-CH=CH=CH₂=, - CH=CH-(CH₂)₂-, -CH=CH-(CH₂)₃-, -CH=CH-(CH₂)₄-, and the like

Specific examples of Y' include -O-(CH₂)₃-, -O-(CH₂)₄-, -O-(CH₂)₅-, -CH₂-O- (CH₂)₂-, -O-CH=CH-CH₂-, S(O)ₘ- (CH₂)₃-(wherein m denotes an integer of 0 to 2), -S(O)ₘ-(CH₂)₄-(wherein m denotes an integer of 0 to 2), -S(O)ₘ-(CH₂)₅-(wherein m denotes an integer of 0 to 2), -CH₂-S(O)ₘ-(CH₂)₂-(wherein m denotes an integer of 0 to 2), -S(O)ₘ-CH=CH-CH₂-(wherein m denotes an integer of 0 to 2), -(CH₂)₄-, -(CH₂)₅-, -(CH₂)₆-, -CH=CH-CH=CH-, -CH=CH-(CH₂)₂-, -NH-(CH₂)₃-, -NH-(CH₂)₄-, -NH-(CH₂)₅-, -CH₂-NH- (CH₂)₂-, -NH-CH=CH-CH₂-, -N=CH-CH=CH-, -CH=N-(CH₂)₂-, -CH=N-CH=CH-, -N=N-(CH₂)₂-, -N=N-CH=CH-, -CH=N-N=CH- (each denoting a bond that starts on ring A), and the like. An 8-membered ring is preferred as ring B.

In addition, the divalent group may have substituent(s), and examples of the substituent include an oxo group and the same substituent as the "substituent" which the "5- to 6-membered ring" of the "optionally substituted 5- to 6-membered ring" represented by R¹ may have. Among them, lower (C₁₋₃) alkyl (e.g., methyl, ethyl, propyl, etc.), phenyl, oxo, a hydroxyl group, and the like are preferred. The substituents of the divalent group may be the same or different, and 1 to 6 (preferably 1 to 2) substituents may be present. They may be present at any positions as far as they can be present.

Examples of the "substituent" which the "8- to 10-membered ring" of the "optionally substituted 8- to 10-membered ring" represented by B may have include an oxo group and the same substituent as the "substituent" which the "5- to 6-membered ring" of the "optionally substituted 5- to 6-membered ring" represented by R¹ may have.

Preferably, the divalent group represented by Y are - O-(CH₂)₃-, -O-(CH₂)₄- -O-(CH₂)₅-, -S(O)ₘ-(CH₂)₃- (m denotes an integer of 0 to 2), -S(O)ₘ-(CH₂)₄- (m denotes an integer of 0 to 2), -S(O)ₘ-(CH₂)₅- (m denotes an integer of 0 to 2), - (CH₂)₄-, -(CH₂)₅, -(CH₂)₆-, and a divalent group represented by the formula -N(R⁰)- (wherein R⁰ denotes a hydrogen atom or a substituent) is present in a primary chain such as - NH-(CH₂)₃-, -NH-(CH₂)₄-, -NH-(CH₂)₅-, or the like.

Preferred examples of R⁰ include a hydrogen atom, an optionally substituted hydrocarbon group, an optionally substituted heterocyclic group, an optionally substituted hydroxyl group, an optionally substituted thiol group (wherein the sulfur atom may be oxidized to form an optionally substituted sulfinyl group or an optionally substituted sulfonyl group), an optionally substituted amino group, an optionally esterified or amidated carboxyl group, an optionally substituted acyl group, and the like, and preferably a hydrogen atom, an optionally substituted hydrocarbon group, an optionally substituted heterocyclic group, an optionally substituted acyl group, and the like.

Preferred embodiments of R⁰ include a hydrogen atom, an optionally substituted hydrocarbon group, an optionally substituted acyl group and the like. As the optionally substituted hydrocarbon group, preferred is optionally halogenated or hydroxylated C₁₋₆ alkyl and optionally halogenated or hydroxylated C₂₋₆ alkenyl. As the optionally substituted acyl group, preferred is optionally halogenated or hydroxylated C₁₋₄ alkylsulfonyl, formyl, optionally halogenated or hydroxylated C₂₋₅ alkanoyl, and the like. More preferably, R⁰ is optionally halogenated or hydroxylated C₁₋₄ alkyl, a formyl, optionally halogenated or hydroxylated C₂₋₅ alkanoyl, and the like, with propyl, isobutyl, isobutenyl, or 3-hydroxy-2-methylpropyl being particularly preferred. Other preferred embodiments of R⁰ include a group represented by the formula -(CH₂)ₛ-R^{x} {wherein s denotes 0 or 1, and R^{x} denotes an optionally substituted 5- to 6-membered aromatic monocyclic group (e.g., the same group as the "5- to 6-membered monocyclic aromatic group" as exemplified with respect to ring A; preferably phenyl, pyrazolyl, thiazolyl, oxazolyl, tetrazolyl, etc., each of which may be substituted with halogen, optionally halogenated or hydroxylated C₁₋₄ alkyl, an optionally halogenated or hydroxylated C₁₋₄ alkoxy, or the like)}.

Examples of the "hydrocarbon group" of the "optionally substituted hydrocarbon group" include:
(1) alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, sec-butyl, tert-butyl, pentyl, isopentyl, neopentyl, hexyl, heptyl, octyl, nonyl, decyl, etc., preferably lower (C₁₋₆) alkyl, and more preferably lower (C₁₋₄) alkyl, or the like);
(2) cycloalkyl (e.g., C₃₋₇ cycloalkyl such as cyclopropyl, cyclobutyl, cyclopentyl, cyclohexyl, cycloheptyl, etc.);
(3) alkenyl (e.g., C₂₋₁₀ alkenyl such as allyl, crotyl, 2-pentenyl, 3-hexenyl, etc., and preferably lower (C₂₋₆) alkenyl, or the like);
(4) cycloalkenyl (e.g., C₃₋₇ cycloalkenyl such as 2-cyclopentenyl, 2-cyclohexenyl, 2-cyclopentenylmethyl, 2-cyclohexenylmethyl, or the like);
(5) alkynyl (e.g., C₂₋₁₀ alkynyl such as ethynyl, 1-propynyl, 2-propynyl, 1-butynyl, 2-pentynyl, 3-hexynyl, etc., preferably lower (C₂₋₆) alkynyl, or the like);
(6) aralkyl (e.g., phenyl-C₁₋₄ alkyl (e.g., benzyl, phenethyl, etc.), or the like);
(7) aryl (e.g., phenyl, naphthyl, or the like);
(8) cycloalkyl-alkyl (e.g., C₃₋₇ cycloalkyl-C₁₋₄ alkyl such as cyclopropylmethyl, cyclobutylmethyl, cyclopentylmethyl, cyclohexylmethyl, cycloheptylmethyl, etc.); and the like. Examples of the substituent which the above (1) alkyl, (2) cycloalkyl, (3)alkenyl, (4) cycloalkenyl, (5) alkynyl, (6) aralkyl, (7) aryl, and (8) cycloalkyl-alkyl may have include halogen, nitro, cyano, hydroxyl, an optionally substituted thiol group (e.g., thiol, C₁₋₄ alkylthio, or the like), an optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, etc., or the like), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, or the like), optionally halogenated C₁₋₄ alkyl (e.g., trifluoromethyl, methyl, ethyl, or the like), optionally halogenated C₁₋₄ alkoxy (e.g., methoxy, ethoxy, propoxy, butoxy, trifluoromethoxy, trifluoroethoxy, or the like), C₁₋₄ alkylenedioxy (e.g., -O-CH₂-O-, -O-CH₂-CH₂-O-, or the like), optionally substituted sulfonamide (e.g., a group formed by the bonding of an optionally substituted amino group (e.g., amino, mono C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., or the like) to -SO₂-, or the like,), formyl, C₂₋₄ alkanoyl (e.g., acetyl, propionyl, or the like), C₁₋₄ alkylsulfonyl (e.g., methanesulfonyl, ethanesulfonyl, or the like), an optionally substituted heterocyclic group, and the like. The number of substituents is preferably 1 to 3.

Examples of the "heterocyclic group" of the "optionally substituted heterocyclic group" and the "optionally substituted heterocyclic group" represented by R⁰ include groups formed by the removal of one hydrogen atom from aromatic heterocyclic ring or non-aromatic heterocyclic rings. Examples of the aromatic heterocyclic ring include a 5- to 6-membered aromatic heterocyclic ring containing 1 to 4 of one or two types of heteroatoms selected from a nitrogen atom, a sulfur atom and an oxygen atom such as furan, thiophene, pyrrole, imidazoles, pyrazole, thiazole, oxazole, isothiazole, triazole, oxadiazole, thiadiazole, and the like. Examples of the non-aromatic heterocyclic ring include a 5- to 6-membered non-aromatic heterocyclic ring containing 1 to 4 heteroatoms of one or two types selected from a nitrogen atom, a sulfur atom, and an oxygen atom such as tetrahydrofuran, tetrahydrothiophene, dioxolane, dithiolane, oxathiolane, pyrrolidone, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, and the like, and a non-aromatic heterocyclic ring in which some or all of the bonds on the aromatic heterocyclic ring are saturated bonds, and the like, preferably an aromatic heterocyclic ring such as pyrazole, thiazole, oxazole, tetrazole, and the like), and the like.

Examples of the "optionally substituted hydroxyl group", the "optionally substituted thiol group", the "optionally substituted amino group", the "optionally esterified carboxyl group," and the "optionally substituted acyl group" represented by R⁰ include the same "optionally substituted hydroxyl group", "optionally substituted thiol group", "optionally substituted amino group", "optionally esterified carboxyl group" and "optionally substituted acyl group" as those which the "5- to 6-membered group" of the "optionally substituted 5- to 6-membered group " represented by R¹ may have as the substituents. Examples of the "optionally amidated carboxyl group" include a group formed by bonding the "optionally substituted amino group", etc. to a carbonyl group, preferably, carbamoyl, mono-C₁₋₆ alkyl-carbamoyl, di-C₁₋₆ alkyl-carbamoyl, and the like.

The imino group optionally substituted with formyl, optionally substituted C₁₋₆ alkyl, optionally substituted C₂-6 alkenyl, optionally substituted aryl, optionally substituted heterocyclic group, optionally substituted aryl methyl, or optionally substituted heterocyclic methyl, which is represented by Y^{a}, denotes a group explained with respect to (R⁰)- represented by Y and fallen within these definitions. Among these groups, preferred is a group wherein R⁰ is (1) C₁₋₆ alkyl, (2) C₂₋₆ alkenyl, (3) C₆₋₁₀ aryl, (4) C₆₋₁₀ aryl-methyl, (5) a heterocyclic group, or (6) a heterocyclic methyl (wherein (1) and (2) may be substituted with halogen or a hydroxyl group, and (3), (4), (5), and (6) may be substituted with halogen, C₁₋₆ alkyl optionally substituted with a hydroxyl group, or a C₁₋₆ alkoxy optionally substituted with halogen or a hydroxyl group).

Further, the substituents of B may be the same or different, and 1 to 7 (preferably 1 to 2) substituents may be present at any positions of the ring (including E₃ and E₄). However, preferably, the E₃ position is unsubstituted.

In the preferred compound represented by the above formula (I), each of E₃ and E₄ is an optionally substituted carbon atom (preferably an unsubstituted carbon atom), and b is a double bond.

In the above formula (I), examples of the "divalent cyclic group" represented by Z¹ include the same group as the 5- to 6-membered ring of the "optionally substituted 5-to 6-membered ring" represented by R¹ or a group formed by removing two hydrogen atoms from a condensed aromatic heterocyclic ring such as benzofuran, indole, benzothiophene, benzoxazole, benzothiazole, indazole, benzimidazole, quinoline, isoquinoline, quinoxaline, phthalazine, quinazoline, cinnoline, imidazopyridine, or the like. Among them, the preferred divalent cyclic group is that formed by removing two hydrogen atoms from benzene, furan, thiophene, pyridine, pyridazine, pyrimidine, benzimidazole, cyclopentane, cyclohexane, pyrrolidone, piperidine, piperazine, morpholine, thiomorpholine, tetrahydropyran, or the like, and the particularly preferred divalent cyclic group is that formed by removing two hydrogen atoms from benzene, pyridine, pyridazine, benzimidazole, cyclohexane, or piperidine (preferably benzene).

The "divalent cyclic group" represented by Z¹ may have the same substituent as that the "5- to 6-membered ring" of the "optionally substituted 5- to 6-membered ring group" represented by R¹ may have. Among them, the preferred substituent include a halogen atom (e.g., fluorine, chlorine, bromine, or the like), C₁₋₄ alkyl group optionally substituted with a halogen atom (e.g., methyl, ethyl, trifluoromethyl, trifluoromethyl, or the like), or C₁₋₄ alkoxy group optionally substituted with a halogen atom (e.g., methoxy, ethoxy, propoxy, trifluoromethoxy, trifluoroethoxy, or the like). However, preferably, the substituents other than X² and Z² are not present. Further, when Z¹ is a 6-membered divalent heterocyclic group (preferably phenylene), the substitution position of Z² is preferably the para-position of X². Furthermore, Z¹ is preferably a phenylene having, as a substituent, 1) a halogen atom, 2) a C₁₋₄ alkyl group optionally substituted with a halogen atom, or 3) a C₁₋₄ alkoxy group optionally substituted with a halogen atom, in particular, a phenylene having, as a substituent, a methyl group or trifluoromethyl group.

The divalent group represented by Z² in the above formula (I) is represented, for example, by the formula - Z^{2a}-W¹-Z^{2b}- (Z^{2a} and Z^{2b} each denote O, S(O)ₘ (wherein m denotes 0, 1, or 2), an optionally substituted imino group (-N (R^{a})-), or a bond, and W¹ denotes an optionally substituted alkylene group, optionally substituted alkenylene group, or a bond). When Z¹ is a benzene ring, for example, the bonding position of Z² may be any position, but is preferably the para-position.

Examples of the substituent (R^{a}) of the optionally substituted imino group represented by Z^{2a} and Z^{2b} include a halogen atom, optionally substituted lower (C₁₋₆) alkyl {e.g., methyl, ethyl, propyl, isopropyl, butyl, isobutyl, sec-butyl, tert-butyl, pentyl, isopentyl, neopentyl, hexyl, hydroxy C₁₋₆ alkyl (e.g., hydroxyethyl, hydroxypropyl, hydroxybutyl, etc.), halogenated C₁₋₆ alkyl (e.g., trifluoromethyl, trifluoromethyl, etc.), cyanated C₁₋₆ alkyl (e.g., cyanoethyl, cyanopropyl, etc.), or optionally esterified or amidated carboxyl C₁₋₆ alkyl}, formyl, lower (C₂₋₅) alkanoyl (e.g., acetyl, propionyl, butyryl, etc.), lower (C₁₋₅) alkylsulfonyl (methylsulfonyl, ethylsulfonyl, etc.), and the like.

Examples of the alkylene group of the "optionally substituted alkylene group" represented by W¹ include an alkylene chain represented by -(CH₂)ₖ₁- (k1 denotes an integer of 1 to 4). Examples of the alkenylene group of the "optionally substituted alkenylene group" represented by W¹ include a alkenylene chain represented by -(CH₂)k₂-(CH=CH)-(CH₂)k₃- (where k2 and k3 are the same or different and denote 0, 1, or 2, and the sum of k2 and k3 is 2 or less).

The alkylene group and the alkenylene group represented by W¹ may have substituent(s) at any position (preferably on a carbon atom), and any substituent may be present as long as it can bond to the alkylene chain or the alkenylene chain that constitutes the linear chain moiety. Examples thereof include lower (C₁₋₆) alkyl (e.g., methyl, ethyl, propyl, isopropyl, butyl, isobutyl, sec-butyl, tert-butyl, pentyl, isopentyl, neopentyl, hexyl, etc.), lower (C₃₋₇) cycloalkyl (e.g., cyclopropyl, cyclobutyl, cyclopentyl, cyclohexyl, cycloheptyl, etc.), formyl, lower (C₂₋₇) alkenoyl (e.g., acetyl, propionyl, butyryl, etc.), an optionally esterified phosphono group, an optionally esterified or amidated carboxyl group, a hydroxyl group, oxo, a hydroxyimino group, an optionally substituted lower (C₁₋₆), alkoxyimino group, and the like, and preferably lower (C₁₋₆) alkyl (preferably C₁₋₃ alkyl), a hydroxyl group, oxo, a hydroxyimino group, a lower (C₁₋₆) alkoxyimino group (which may be substituted with a polar group such as a hydroxyl group, cyano group, an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), etc.), and the like.

As the optionally esterified phosphono group, there is a group represented by P(O) (OR⁹) (OR¹⁰) (wherein, R⁹ and R¹⁰ each denote a hydrogen atom, a C₁₋₆ alkyl group, or a C₃₋₇ cycloalkyl group, or R⁹ and R¹⁰ may bond to each other to form a 5- to 7-membered ring).

In the above formula, examples of the C₁₋₆ alkyl group represented by R⁹ and R¹⁰ include methyl, ethyl, propyl, isopropyl, butyl, isobutyl, sec-butyl, tert-butyl, pentyl, isopentyl, neopentyl, hexyl, etc., and examples of the C₃₋₇ cycloalkyl include cyclopropyl, cyclobutyl, cyclopentyl, cyclohexyl, cycloheptyl, etc. However, the preferred group is linear lower (C₁₋₆) alkyl, and lower (C₁₋₃) alkyl is more preferred. R⁹ and R¹⁰ may be the same or different, preferably the same. In addition, when R⁹ and R¹⁰ bond to each other to form a 5- to 7-membered ring, R⁹ and R¹⁰ bond to each other to form a linear C₂₋₄ alkylene side chain represented by - (CH₂)₂-, -(CH₂)₃-, or -(CH₂)₄-. The side chain may have substituent(s), and examples of the substituent include a hydroxyl group, halogen, and the like.

Examples of the ester form of the optionally esterified carboxyl group include an ester formed by bonding a carboxyl group to a C₁₋₆ alkyl group or a C₃₋₇ cycloalkyl group, for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, cyclopentyloxycarbonyl, cyclohexyloxycarbonyl, and the like.

Examples of the amide form of the optionally amidated carboxyl group include an amide formed by bonding a carboxyl group to a C₁₋₆ alkylamino group, a C₃₋₇ cycloalkylamino group, or a 5- to 8-membered cyclic amine (e.g., pyrrolidine, piperidine, morpholine, etc.), for example, carbamoyl, mono-C₁₋₆ alkylcarbamoyl, di-C₁₋₆ alkylcarbamoyl, cyclopentylaminocarbonyl, cyclohexylaminocarbonyl, pyrrolidinocarbonyl, piperidinocarbonyl, morpholinocarbonyl, thiomorpholinocarbonyl, and the like.

As Z², a preferred group is a divalent group wherein either one of Z^{2a} or Z^{2b} is O, S(O)ₘ (m is 0, 1, or 2), or - N(R^{a})- (wherein R^{a} denotes a hydrogen atom or an optionally substituted lower C₁₋₄ alkyl group), and the other is a bond, and W is -(CH₂)ₚ- (wherein p denotes an integer of 1 to 3), or Z² is preferably -CH(OH)-. More preferably, Z² is a divalent group wherein either one of Z^{2a} or Z^{2b} is O or S(O)ₘ (m is 0, 1, or 2) and the other is a bond, and W is - (CH₂)ₚ- (where p denotes an integer of 1 to 3) or Z² is - CH(OH)-. Further more preferably, Z² is -CH₂-, -CH(OH)- or -S(O)ₘ-CH₂ (where m denotes 0, 1, or 2), with -S(O)ₘ-CH₂-(m is 0, 1, or 2) being particularly preferred. In particular, when Z^{2a} is bonded to Z¹, -SOCH₂- is preferred.

Z^{2a} denotes a bond, S, SO, or SO₂. Among them, SO is preferred, and in this case, the configuration of SO is preferably (S).

Examples of the "optionally substituted amino group wherein the nitrogen atom may be converted to a quaternary ammonium or oxide" in the above formula (I) include an amino group that may have 1 to 2 substituents, an amino group having three substituents wherein the nitrogen atom has been converted to a quaternary ammonium group, and the like. When the number of substituents on the nitrogen atom is 2 or more, these substituents may be the same or different, and when the number of substituents on the nitrogen atom is 3, the amino group may be any type of - N⁺R^{p}R^{p}R^{p}, -N⁺R^{p}R^{p}R^{q}, and -N⁺R^{p}R^{q}R^{r} (where R^{p}, R^{q}, and R^{r} are different and each denote a hydrogen atom or a substituent). Further, examples of the counter ion of the amino group wherein the nitrogen atom has been converted to a quaternary ammonium group include, in addition to an anion of a halogen atom (e.g., Cl⁻, Br⁻, I⁻ or the like), anions derived from inorganic acids such as hydrochloric acid, hydrobromic acid, nitric acid, sulfuric acid, phosphoric acid, etc., anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc., anions derived from acidic amino acid such as aspartic acid, glutamic acid, etc., and the like, with Cl⁻, Br⁻ and I⁻ being preferred.

Examples of the substituent of amino group include:
(1) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, or the like);
(2) optionally substituted cycloalkyl (e.g., C₃₋₈ cycloalkyl such as cyclopropyl, cyclobutyl, cyclopentyl, cyclohexyl, cycloheptyl, cyanooctyl, or the like);
   (2-1) the above cycloalkyl may contain one heteroatom selected from a sulfur atom, an oxygen atom, and a nitrogen atom, may form oxirane, thiolane, aziridine, tetrahydrofuran, tetrahydrothiophene, pyrrolidine, tetrahydropyran, tetrahydrothiopyran, tetrahydrothiopyran, 1-oxide, piperidine, etc. (preferably a 6-membered ring such as tetrahydropyran, tetrahydrothiopyran, piperidine, etc.), and the like, and preferably bonds to the amino group at the 3-position or 4-position (preferably the 4-position);
   (2-2) further, the cycloalkyl may be condensed with a benzene ring to form an indane (e.g., indan-1-yl, indan-2-yl, etc.), tetrahydronaphthalene (e.g., tetrahydronaphthalen-5-yl, tetrahydronaphthalen-6-yl, etc.), and the like (preferably indane, or the like);
   (2-3) furthermore, the cycloalkyl may be cross-linked via a linear atom chain having 1 to 2 carbon atoms to form a crosslinked cyclic hydrocarbon residue such as bicyclo[2.2.1]heptyl, bicyclo[2.2.2]octyl, bicyclo[3.2.1]octyl, bicyclo[3.2.2], and the like (preferably a crosslinked cyclohexyl having a cross-link of a linear atomic chain of 1 to 2 carbon atoms, and more preferably bicyclo[2.2.1]heptyl, and the like);
(3) optionally substituted alkenyl (e.g., C₂₋₁₀ alkenyl such as allyl, crotyl, 2-pentenyl, 3-hexenyl, etc., preferably lower (C₂₋₆) alkenyl, or the like);
(4) optionally substituted cycloalkenyl (e.g., C₃₋₇ cycloalkenyl such as 2-cyclopentenyl, 2-cyclohexenyl, 2-cyclopentenylmethyl, 2-cyclohexenylmethyl, etc., or the like);
(5) optionally substituted aralkyl (e.g., phenyl-C₁₋₄ alkyl (e.g., benzyl, phenethyl, or the like);
(6) formyl or optionally substituted acyl (e.g., C₂₋₄ alkanoyl (e.g., acetyl, propionyl, butyryl, isobutyryl, etc.), C₁₋₄ alkylsulfonyl (e.g., methanesulfonyl, ethanesulfonyl, etc.), C₁₋₄ alkoxy-carbonyl with (e.g., methoxycarbonyl, ethoxycarbonyl, tert-butoxycarbonyl, etc.), or C₇₋₁₀ aralkyloxycarbonyl (e.g., benzyloxy, carbonyl, etc.), or the like);
(7) optionally substituted aryl (e.g., phenyl, naphthyl, or the like);
(8) optionally substituted heterocyclic group (e.g., a group formed by removing one hydrogen atom from a 5- to 6-membered aromatic heterocyclic ring containing 1 to 4 heteroatoms of 1 or 2 types selected from a nitrogen atom, a sulfur atom, and an oxygen atom such as a furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, etc.; a group formed by removing one hydrogen atom from a condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; or a group formed by removing one hydrogen atom from a 5- to 6-membered non-aromatic heterocyclic ring containing 1 to 4 heteroatoms of one or two types selected from a nitrogen atom, a sulfur atom, and an oxygen atom, such as tetrahydrofuran, tetrahydrothiophene, dithiolane, oxathiolane, pyrrolidone, pyrroline, imidazolidine, imidazoline, pyrazolidine, pyrazoline, piperidine, piperazine, oxazine, oxadiazine, thiazine, thiadiazine, morpholine, thiomorpholine, pyran tetrahydropyran, etc.; preferably a group formed by removing one hydrogen atom from a 5- to 6-membered non-aromatic heterocyclic ring, or the like; more preferably a group formed by removing one hydrogen atom from a 5- to 6-membered non-aromatic heterocyclic ring containing one heteroatom such as tetrahydrofuran, piperidine, tetrahydropyran, tetrahydrothiopyran, etc.), and the like. Further, the substituents of the amino group may bond to each other to form 5- to 7-membered cyclic amino such as piperidine, piperazine, morpholine, thiomorpholine, and the like.

Examples of the substituent which the (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, (7) optionally substituted aryl, and (8) optionally substituted heterocyclic group may have include halogen, optionally halogenated lower (C₁₋₄) alkyl, lower (C₁₋₄) alkyl optionally substituted with a polar group such as a hydroxyl group, a cyano group, an optionally esterified or amidated carboxyl group etc. (e.g., hydroxy C₁₋₄ alkyl, cyano C₁₋₄ alkyl, carboxyl C₁₋₄ alkyl, C₁₋₄ alkoxycarbonyl, C₁₋₄ alkyl, carbamoyl C₁₋₄ alkyl, mono-C₁₋₄ alkylcarbamoyl, C₁₋₄ alkyl, di-C₁₋₄ alkylcarbamoyl, di-C₁₋₄ alkylcarbamoyl C₁₋₄ alkyl, pyrrolidinocarbonyl C₁₋₄ alkyl, piperidinocarbonyl C₁₋₄ alkyl, morpholinocarbonyl C₁₋₄ alkyl, thiomorpholinocarbonyl C₁₋₄ alkyl, etc.), optionally halogenated C₁₋₄ alkoxy (e.g., methoxy, ethoxy, propoxy, butoxy, trifluoromethoxy, trifluoromethoxy, etc.), C₁₋₄ alkylenedioxy (e.g., -O-CH₂-O-, -O-CH₂-CH₂-O-, etc.), formyl, C₂₋₄ alkanoyl (e.g., acetyl,, propionyl, etc.), C₁₋₄ alkylsulfonyl (e.g., methanesulfonyl, ethanesulfonyl, etc.), phenyl-lower (C₁₋₄) alkyl, C₃₋₇ cycloalkyl, cyano, nitro, hydroxyl group, optionally substituted thiol group (e.g., thiol, C₁₋₄ alkylthio, etc.), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc.), optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), lower (C₁₋₄) alkoxy-carbonyl, lower (C₇₋₁₀) aralkyloxycarbonyl, oxo group (preferably halogen, optionally halogenated lower (C₁₋₄) alkyl, optionally halogenated lower (C₁₋₄) alkoxy, phenyl-lower (C₁₋₄) alkyl, C₃₋₇ cycloalkyl, cyano, hydroxyl group, etc.), and the like. The number of substituents is preferably 1 to 3.

The "optionally substituted amino group wherein the nitrogen atom is converted to a quaternary ammonium or an oxide" represented by R² in the above formula (I) is preferably an amino group having 1 to 3 substituents selected from:
(1) linear or branched lower (C₁₋₆) alkyl which may have 1 to 3 substituents selected from halogens, cyanos, hydroxyl groups and C₃₋₇ cycloalkyls;
(2) C₅₋₈ cycloalkyl which may have 1 to 3 substituents selected from halogens, optionally halogenated lower (C₁₋₄) alkyls and phenyl-lower (C₁₋₄) alkyls, may contain one heteroatom selected from a sulfur atom, an oxygen atom and a nitrogen atom, may be condensed with a benzene ring, and may be cross-linked via a linear chain having 1 to 2 carbon atoms (e.g., cyclopentyl, cyclohexyl, cycloheptyl, cyclooctyl, tetrahydropyranyl, tetrahydrothiopyranyl, piperidinyl, indanyl, tetrahydronaphthalenyl, bicyclo[2.2.1]heptyl, etc., each of which may be substituted);
(3) phenyl-lower (C₁₋₄) alkyl which may have 1 to 3 substituents selected from halogens, optionally halogenated lower (C₁₋₄) alkyls and optionally halogenated lower (C₁₋₄) alkoxys;
(4) phenyl which may have 1 to 3 substituents selected from halogens, optionally halogenated lower (C₁₋₄) alkyls, and optionally halogenated lower (C₁₋₄) alkoxys; and
(5) a 5- to 6-membered aromatic heterocyclic group which may have 1 to 3 substituents selected from halogens, optionally halogenated lower (C₁₋₄) alkyl groups, optionally halogenated lower (C₁₋₄) alkoxys, optionally halogenated lower (C₁₋₄) alkoxy-lower (C₁₋₄) alkoxys, phenyl-lower (C₁₋₄) alkyls, cyanos, and hydroxyls (e.g., a group formed by removing one hydrogen atom from furan, thiophene, pyrrole, pyridine, etc.).

Examples of the "nitrogen-containing heterocyclic ring" of the "optionally substituted nitrogen-containing heterocyclic ring which may contain a sulfur atom or an oxygen atom as a ring-forming atom and wherein the nitrogen atom may be converted into a quaternary ammonium or oxide" represented by R² in the above formula (I) include a 5- to 6-membered aromatic heterocyclic ring containing 1 to 4 heteroatoms of 1 or 2 type selected from a nitrogen atom, a sulfur atom, and an oxygen atom such as pyrrole, imidazole, pyrazole, thiazole, oxazole, isothiazole, isoxazole, tetrazole, pyridine, pyrazine, pyrimidine, pyridazine, triazole, oxadiazole, thiadiazole, etc. ; a condensed aromatic heterocyclic ring such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; a 5- to 8-membered non-aromatic heterocyclic ring which may have, in addition to one nitrogen atom, 1 to 3 heteroatoms of 1 or 2 types selected from a nitrogen atom, a sulfur atom and an oxygen atom such as pyrrolidone, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacyclooctane (azocaine), etc.; and the like, wherein these nitrogen-containing heterocyclic rings may be cross-linked via a linear atomic chain having 1 to 2 carbon atoms to form crosslinked cyclic nitrogen-containing heterocyclic rings such as azabicyclo[2.2.1]heptane, azabicyclo[2.2.2]octane (quinacridine), and the like (preferably piperidine cross-linked via a linear chain having 1 to 2 carbon atoms, and the like).

Among the abovementioned specific examples of the nitrogen-containing heterocyclic rings, preferred are pyridine, pyridazine, pyrazole, imidazoles, triazole, tetrazole, imidazopyridine, pyrrolidone, piperidine, piperazine, morpholine, thiomorpholine, and azabicyclo[2.2.2]octane (preferably pyridine, imidazoles, triazole, imidazopyridine, pyrrolidone, piperidine and morpholine).

The nitrogen atom of the "nitrogen-containing heterocyclic ring" may be converted to a quaternary ammonium or may be oxidized. When the nitrogen atom of the "nitrogen-containing heterocyclic ring" is converted to a quaternary ammonium, examples of the counter ion for the "nitrogen-containing heterocyclic ring wherein the nitrogen atom is converted to a quaternary ammonium" include, in addition to anions of halogen atoms (e.g., Cl⁻, Br⁻, and I⁻), anions derived from inorganic acid such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; anions derived from acidic amino acid such as aspartic acid, glutamic acid, etc.; and the like, with Cl⁻, Br⁻, and I⁻ being particularly preferred.

The "nitrogen-containing heterocyclic ring" may be bonded to the divalent group represented by Z² via either a nitrogen atom or a carbon atom, or may be bonded to a ring-forming carbon atom such as with 2-pyridyl, 3-pyridyl, 2-piperidinyl, and the like. Preferably, it may be bonded to a ring-forming nitrogen atom such as: 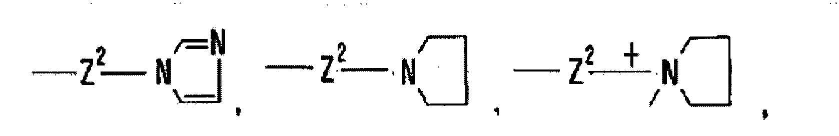 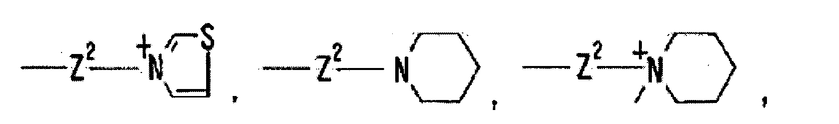 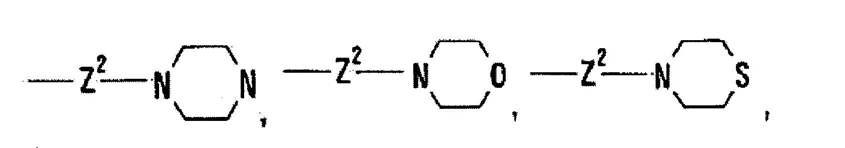 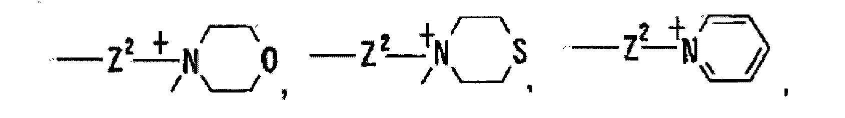 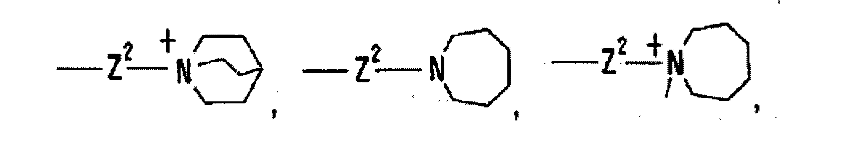 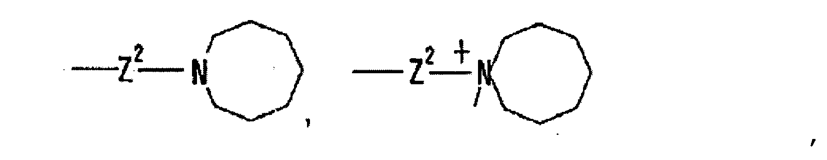 and the like.

Examples of the substituent which the "nitrogen-containing heterocyclic ring" may have include halogen, optionally substituted lower (C₁₋₄) alkyl, optionally substituted lower (C₁₋₄) alkoxy, optionally substituted phenyl, optionally substituted mono- or di-phenyl-lower (C₁₋₄) alkyl, optionally substituted C₃₋₇ cycloalkyl, cyano, nitro, a hydroxyl group, an optionally substituted thiol group (e.g., thiol, C₁₋₄ alkylthio, etc.), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., or the like), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), lower (C₁₋₄) alkoxy-carbonyl, formyl, lower (C₂₋₄) alkanoyl, lower (C₁₋₄) alkylsulfonyl, an optionally substituted heterocyclic group (e.g., a group formed by removing one hydrogen atom from a 5- to 6-membered aromatic hetero ring containing 1 to 4 heteroatoms of 1 or 2 types selected from a nitrogen atom, a sulfur atom and an oxygen atom such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, etc.; a group formed by removing one hydrogen atom from a condensed aromatic heterocyclic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; a group formed by removing one hydrogen atom from a 5- to 6-membered non-aromatic hetero ring containing 1 to 4 heteroatoms of one or two types selected from a nitrogen atom, a sulfur atom and an oxygen atom such as tetrahydrofuran, tetrahydrothiophene, dithiolane, oxathiolane, pyrrolidone, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.; or the like), and the like. The number of the substituents is 1 to 3. In addition, the nitrogen atom of the "nitrogen-containing heterocyclic ring" may be oxidized.

Examples of the substituent which the "optionally substituted lower (C₁₋₄) alkyl", the "optionally substituted lower (C₁₋₄) alkoxy", the "optionally substituted phenyl", the "optionally substituted mono- or di-phenyl lower (C₁₋₄) alkyl", the "optionally substituted C₃₋₇ cycloalkyl", and the "optionally substituted heterocyclic group" as the substituents of the "nitrogen-containing heterocyclic ring" may have include a halogen atom (e.g., fluorine, chlorine, bromine, iodine, etc.), optionally halogenated lower (C₁₋₄) alkyl, lower (C₁₋₄) alkyl optionally substituted with a polar group such as a hydroxyl group, a cyano group, an optionally esterified or amidated carboxyl group (e.g., hydroxy C₁₋₄ alkyl, cyano C₁₋₄ alkyl, carboxyl C₁₋₄ alkyl, C₁₋₄ alkoxy-carbonyl C₁₋₄ alkyl, carbamoyl C₁₋₄ alkyl, mono-C₁₋₄ alkyl-carbamoyl C₁₋₄ alkyl, di-C₁₋₄ alkyl-carbamoyl C₁₋₄ alkyl, pyrrolidinocarbonyl C₁₋₄ alkyl, piperidinocarbonyl C₁₋₄ alkyl, morpholinocarbonyl C₁₋₄ alkyl, thiomorpholinocarbonyl C₁₋₄ alkyl, etc.), lower (C₃₋₁₀) cycloalkyl, lower (C₃₋₁₀) cycloalkenyl, optionally halogenated C₁₋₄ alkoxy (e.g., methoxy, ethoxy, trifluoromethoxy, trifluoroethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), C₁₋₃ alkylenedioxy (e.g., methylenedioxy, ethylenedioxy, etc.), cyano, nitro, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), lower (C₁₋₄) alkoxy-carbonyl, and the like. The number of the substituents is preferably 1 to 3.

Preferred examples of the substituent which the "nitrogen-containing heterocyclic ring" of the "optionally substituted nitrogen-containing heterocyclic group which may contain a sulfur atom or an oxygen atom as a ring-forming atom and wherein the nitrogen atom may be converted into a quaternary ammonium or oxide" in the above formula (I) include (1) halogen, (2) cyano, (3) a hydroxyl group, (4) a carboxyl group, (5) a carbamoyl group, (6) lower (C₁-₄) alkoxycarbonyl, (7) lower (C₁₋₄) alkylcarbamoyl or 5- to 6-membered amino (piperidino, morpholino, etc.)-carbonyl, (8) lower (C₁₋₄) alkyl optionally substituted with halogen, a hydroxyl group, a cyano group, lower (C₁₋₄) alkoxy, or an optionally esterified or amidated carboxyl group, (9) lower (C₁₋₄) alkoxy optionally substituted with halogen, a hydroxyl group or lower (C₁₋₄) alkoxy, (10) phenyl optionally substituted with halogen, lower (C₁₋₄) alkyl, a hydroxyl group, lower (C₁₋₄) alkoxy, or C₁₋₃ alkylenedioxy, (11) mono- or diphenyl-lower (C₁₋₄) alkyl optionally substituted with halogen, lower (C₁₋₄) alkyl, a hydroxyl group, lower (C₁₋₄) alkoxy, or (C₁₋₃) alkylenedioxy, (12) a group formed by removing one hydrogen atom from a 5- to 6-membered aromatic heterocyclic ring such as furan, thiophene, pyrrole, pyridine, etc., and the like.

Examples of the "optionally substituted hydrocarbon group" represented by R⁵ and R⁶ in the "group represented by the formula: 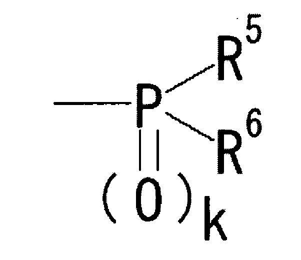 wherein k denotes 0 or 1, and when k is 0, the phosphorus atom can form a phosphonium salt; R⁵ and R⁶ each denote an optionally substituted hydrocarbon group, an optionally substituted hydroxyl group, or an optionally substituted amino group (preferably, an optionally substituted hydrocarbon group or an optionally substituted amino group, more preferably, an optionally substituted hydrocarbon group); or R⁵ and R⁶ may bond to each other to form a cyclic group together with the adjacent phosphorus atom" represented by R² in the above formula (I) include:
(1) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl, or the like);
(2) optionally substituted cycloalkyl (e.g., C₃₋₇ cycloalkyl such as cyclopropyl, cyclobutyl, cyclopentyl, cyclohexyl, cycloheptyl, or the like);
(3) optionally substituted alkenyl (e.g., C₂₋₁₀ alkenyl such as allyl, crotyl, 2-pentenyl, 3-hexenyl, etc., and preferably lower (C₂₋₆) alkenyl, or the like);
(4) optionally substituted cycloalkenyl (e.g., C₃₋₇ cycloalkenyl such as 2-cyclopentenyl, 2-cyclohexenyl, 2-cyclopentenylmethyl, 2-cyclohexenylmethyl, etc.);
(5) optionally substituted alkynyl (e.g., C₂₋₁₀ alkynyl such as ethynyl, 1-propynyl, 2-propynyl, 1-butynyl, 2-pentynyl, 3-hexynyl, etc., preferably lower (C₂₋₆) alkynyl, or the like);
(6) optionally substituted aralkyl (e.g., phenyl-C₁₋₄ alkyl (e.g., benzyl, phenethyl, etc.), or the like);
(7) optionally substituted aryl (e.g., phenyl, naphthyl, etc.), and the like. Examples of the substituent which the above (1) optionally substituted alkyl, (2) optionally substituted cycloalkyl, (3) optionally substituted alkenyl, (4) optionally substituted cycloalkenyl, (5) optionally substituted alkynyl, (6) optionally substituted aralkyl, and (7) optionally substituted aryl may have include halogen, nitro, cyano, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkyl (e.g., trifluoromethyl, methyl, ethyl, etc.), optionally halogenated C₁₋₄ alkoxy (e.g., methoxy, ethoxy, trifluoromethoxy, trifluoromethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like. The number of the substituents is preferably 1 to 3.

Examples of the "optionally substituted hydroxyl group" represented by R⁵ and R⁶ include a hydroxyl group which may have:
(1) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, or the like);
(2) optionally substituted cycloalkyl (e.g., C₃₋₇ cycloalkyl such as cyclopropyl, cyclobutyl, cyclopentyl, cyclohexyl, cycloheptyl, or the like),
(3) optionally substituted alkenyl (e.g., C₂₋₁₀ alkenyl such as allyl, crotyl, 2-pentenyl, 3-hexenyl, etc., preferably lower (C₂₋₆) alkenyl, or the like);
(4) optionally substituted cycloalkenyl (e.g., C₃₋₇ cycloalkenyl such as 2-cyclopentenyl, 2-cyclohexenyl, 2-cyclopentenylmethyl, 2-cyclohexenylmethyl, or the like);
(5) optionally substituted aralkyl (e.g., phenyl-C₁₋₄ alkyl (e.g., benzyl or phenethyl, etc.), or the like);
(6) formyl or optionally substituted acyl (e.g., C₂₋₄ alkanoyl (e.g., acetyl, propionyl, butyryl, isobutyryl, etc.), C₁₋₄ alkylsulfonyl (e.g., methanesulfonyl, ethanesulfonyl, etc.), or the like);
(7) optionally substituted aryl (e.g., phenyl, naphthyl, etc.), and the like.

Examples of the substituent which the above (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl and (7) optionally substituted aryl include halogen, nitro, cyano, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., or the like), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkyl (e.g., trifluoromethyl, methyl, ethyl, etc.), optionally halogenated C₁₋₄ alkoxy (e.g., methoxy, ethoxy, trifluoromethoxy, trifluoroethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like. The number of the substituents is preferably 1 to 3.

In the above formula (I), examples of the counter anion when the phosphorus atom forms a phosphonium salt include, in addition to anions of halogen atoms (e.g., Cl⁻, Br⁻, and I⁻), anions derived from inorganic acid such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; anions derived from acidic amino acid such as aspartic acid, glutamic acid, etc.; and the like, with Cl⁻, Br⁻, and I⁻ being preferred.

Examples of the optionally substituted amino group represented by R⁵ and R⁶ include an amino group having 1 or 2 of:
(1) optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, or the like);
(2) optionally substituted cycloalkyl (e.g., C₃₋₇ cycloalkyl such as cyclopropyl, cyclobutyl, cyclopentyl, cyclohexyl, cycloheptyl, or the like),
(3) optionally substituted alkenyl (e.g., C₂₋₁₀ alkenyl such as allyl, crotyl, 2-pentenyl, 3-hexenyl, etc., preferably lower (C₂₋₆) alkenyl, or the like);
(4) optionally substituted cycloalkenyl (e.g., C₃₋₇ cycloalkenyl such as 2-cyclopentenyl, 2-cyclohexenyl, 2-cyclopentenylmethyl, 2-cyclohexenylmethyl, or the like);
(5) formyl or optionally substituted acyl (e.g., C₂₋₄ alkanoyl (e.g., acetyl, propionyl, butyryl, isobutyryl, etc.), or C₁₋₄ alkylsulfonyl (e.g., methanesulfonyl, ethanesulfonyl, etc.) or the like);
(6) optionally substituted aryl (e.g., phenyl, naphthyl, etc.), and the like.

Examples of the substituent which the above (1) optionally substituted alkyl, (2) optionally substituted cycloalkyl, (3) optionally substituted alkenyl, (4) optionally substituted cycloalkenyl, (5) optionally substituted acyl, and (6) optionally substituted aryl may have include halogen, nitro, cyano, a hydroxyl group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc.), an optionally esterified or amidated carboxyl group (e.g., carboxyl, C₁₋₄ alkoxy-carbonyl, carbamoyl, mono-C₁₋₄ alkyl-carbamoyl, di-C₁₋₄ alkyl-carbamoyl, etc.), optionally halogenated C₁₋₄ alkyl (e.g., trifluoromethyl, methyl, ethyl, etc.), optionally halogenated C₁₋₄ alkoxy (e.g., methoxy, ethoxy, trifluoromethoxy, trifluoroethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), and the like. The number of the substituents is preferably 1 to 3.

Examples of the substituent of the "optionally substituted guanidine group" and the "optionally substituted amidino group" represented by R² include the same as those of the above "optionally substituted amino group wherein the nitrogen atom may be converted into a quaternary ammonium or oxide" represented by R².

Preferably, R² is (1) an optionally substituted amino group where the nitrogen atom may be converted into a quaternary ammonium or oxide, (2) an optionally substituted nitrogen-containing heterocyclic group which may contain a sulfur atom or an oxygen atom as a ring-forming atom, and wherein the nitrogen atom may be converted into a quaternary ammonium or oxide, (3) an optionally substituted amidino group, or (4) an optionally substituted guanidino group. More preferably, R² is an optionally substituted amino group wherein the nitrogen atom may be converted into a quaternary ammonium or oxide, an optionally substituted nitrogen-containing heterocyclic group which may contain a sulfur atom or an oxygen atom as a ring-forming atom, and wherein the nitrogen atom may be converted into an oxide, and the like. In particular, preferred are an optionally substituted nitrogen-containing heterocyclic group which may contain an oxygen atom or a sulfur atom as a ring-forming atom, and the like.

Furthermore, R² is preferably a group represented by the formula -NRR" or -N+RR'R " (wherein R, R', and R" each denote an optionally substituted aliphatic hydrocarbon group (an aliphatic chain-form hydrocarbon group or an aliphatic cyclic hydrocarbon group), or an optionally substituted alicyclic (non-aromatic) heterocyclic group), or an optionally substituted nitrogen-containing aromatic heterocyclic group wherein the nitrogen atom may be oxidized.

Examples of the "optionally substituted alicyclic heterocyclic group" and the "optionally substituted aliphatic hydrocarbon group" represented by R, R', and R" in the above formula include the same groups as the "optionally substituted aliphatic hydrocarbon group" (e.g., alkyl, cycloalkyl, alkenyl, cycloalkenyl, etc., each of which may be substituted) and the "optionally substituted alicyclic heterocyclic group" (e.g., 5- to 6-membered optionally substituted non-aromatic heterocyclic ring, etc.) as exemplified with respect to the substituent which the "optionally substituted amino group" represented by substituent R² may have.

Among them, preferred R and R' are an optionally substituted chain-form hydrocarbon group (e.g., alkyl, alkenyl, etc., each of which may be substituted), and an optionally substituted C₁₋₆ alkyl group is more preferred with an optionally substituted methyl group being particularly preferred.

Preferably, R" is an optionally substituted alicyclic hydrocarbon group (preferably, an optionally substituted C₃₋₈ cycloalkyl group; more preferably an optionally substituted cyclohexyl group) or an optionally substituted alicyclic heterocyclic group (preferably an optionally substituted saturated alicyclic heterocyclic group (preferably a 6-membered cyclic group). More preferably, R" is an optionally substituted tetrahydropyranyl, optionally substituted tetrahydrothiopyranyl, or optionally substituted piperidyl, with an optionally substituted tetrahydropyranyl being particularly preferred.

Further, as the "nitrogen-containing aromatic heterocyclic groups" of the "optionally substituted nitrogen-containing aromatic heterocyclic group wherein the nitrogen atom may be oxidized" represented by R², imidazole or triazole is particularly preferred, among the exemplified preferred groups, pyridine, imidazole, triazole, and imidazopyridine.

Examples of the "optionally substituted amino group wherein the nitrogen atom may be converted into a quaternary ammonium or oxide" represented by R^{2'} and R^{2"} and the like include the same as the corresponding group of the above R².

Examples of the substituent represented by R⁴ of the imino group of Y and the "optionally substituted hydrocarbon group" and the "optionally substituted C₁₋₆ alkyl" in the substituent of the imino group of Y' include the same as the corresponding groups of the above R⁰.

Examples of the "optionally substituted alkylene chain" of W² include the same as the corresponding group of W¹_{.}

Specific examples of the preferred compound represented by formula (I) include the following compounds and salts.
8-[4-(2-Butoxyethoxy)phenyl]-N-[4-[[N-methyl-N-(tetahydropyran-4-yl)amino]methyl]phenyl]-3,4-dihydro-2H-1-benzoxocin-5-carboxamide;
8-[4-(2-Butoxyethoxy)phenyl]-N-[4-[[N-methyl-N-(tetahydropyran-4-yl)amino]methyl]phenyl]-1,2,3,4-tetrahydro-1-benzazocine-5-carboxamide;
8-[4-(2-Butoxyethoxy)phenyl]-1-propyl-N-[4-[[[1-propylimidazol-5-yl]methyl]sulfanyl]phenyl]-1,2,3,4-tetrahydro-1-benzazocine-5-carboxamide;
8-[4-(2-Butoxyethoxy)phenyl]-1-propyl-N-[4-[[[1-propylimidazol-5-yl]methyl]sulfinyl]phenyl]-1,2,3,4-tetrahydro-1-benzazocine-5-carboxamide;
8-[4-(2-Butoxyethoxy)phenyl]-1-propyl-N-[4-[[[1-propylimidazol-5-yl]methyl]sulfonyl]phenyl]-1,2,3,4-tetrahydro-1-benzazocine-5-carboxamide;
8-[4-(2-Butoxyethoxy)phenyl]-1-isobutyl-N-[4-[[[1-propylimidazol-5-yl]methyl]sulfanyl]phenyl]-1,2,3,4-tetrahydro-1-benzazocine-5-carboxamide ;
8-[4-(2-Butoxyethoxy)phenyl]-1-isobutyl-N-[4-[[[1-propylimidazol-5-yl]methyl]sulfinyl]phenyl]-1,2,3,4-tetrahydro-1-benzazocine-5-carboxamide ;
8-[4-(2-Butoxyethoxy)phenyl]-1-isobutyl-N-[4-[[[1-propylimidazol-5-yl]methyl]sulfonyl]phenyl]-1,2,3,4-tetrahydro-1-benzazocine-5-carboxamide;
8-[4-(2-Butoxyethoxy)phenyl]-1-isobutyl-N-[4-[[N-methyl-N-(tetrahydropyran-4-yl)amino]methyl]phenyl]-1,2,3,4-tetrahydro-1-benzazocine-5-carboxamide;
(S)-8-[4-(2-Butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide methanesulfonate;
(S)-8-[4-(2-Butoxyethoxy)phenyl]-1-propyl-N-[4-[[(1-propyl-1H-imidazole-5-yl)methyl]sulfinyl]phenyl]-1,2,3,4-tetrahydro-1-benzazocine-5-carboxamide methanesulfonate;
(S)-1-isobutyl-8-[4-(2-propoxyethoxy)phenyl]-N-[4-[[(1-propyl-1H-imidazol-5-yl)methyl]sulfinyl]phenyl]-1,2,3,4-tetrahydro-1-benzazocine-5-carboxamide;
(S)-8-[4-(2-Butoxyethoxy)phenyl]-1-[(1-methyl-1H-pyrazol-4-yl)methyl]-N-[4-[[(1-propyl-1H-imidazol-5-yl)methyl]sulfinyl]phenyl]-1,2,3,4-tetrahydro-1-benzazocine-5-carboxamide; and
(S)-8-[4-(2-butoxyethoxy)phenyl]-1-isobutyl-N-[4-[[(4-propyl-4H-1,2,4-triazol-3-yl)methyl]sulfinyl]phenyl]-1,2,3,4-tetrahydro-1-benzazocine-5-carboxamide.

The surface modifier used in the present invention is an agent which improves the stability of a base drug, a disintegration rate and compression properties by covering the surface of a base drug, an acid or a base. Examples thereof include colloidal silicon dioxide (e.g., Aerosil, manufactured by Japan Aerosil), calcium silicate, aluminum silicate, titanium dioxide, and the like.

Compounds that are extremely poorly soluble in their free form include amphoteric, basic, and acidic compounds. When the compound is an amphoteric or basic compound, an acid is blended in order to achieve the objects of the present invention, whereas a base is blended when the compound is an amphoteric or acidic compound.

The terms "amphoteric compound," "basic compound," and "acidic compound" used herein have common meanings. Specifically, for example, it can be defined based on the pKa value (a logarithm of a reciprocal of an acid dissociation constant) of the partial structure of the compound. At this time, the term "amphoteric compound" refers to a compound having a partial structure exhibiting a pKa value of 7.5 or more (preferably 8.5 or more) and having a partial structure exhibiting a pKa value of 6.5 or less (preferably 5.5 or less). The term "basic compound" refers to a compound having a partial structure exhibiting a pKa of 7.5 or more (preferably 8.5 or more). The term "acidic compound" refers to a compound having a partial structure exhibiting a pKa of 6.5 or less (preferably 5.5 or less).

Examples of the acid used in the present invention include any acid which is a solid or a liquid at ordinary temperature (15 to 25°C), but a solid acidic compound is preferably used. Examples of the "acid" include carboxylic acids (e.g., acetic acid, lactic acid, fumaric acid, oxalic acid, succinic acid, citric acid (including citric acid anhydrate), oxalic acid, malonic acid, maleic acid, dl-malic acid, stearic acid, adipic acid), sulfonic acids (e.g., aminoethylsulfonic acid), acidic polysaccharides (e.g., alginic acid), acidic amino acids (e.g., glutamic acid, aspartic acid), salts of inorganic acids with amino acids (e.g., glycine hydrochloride, aspartic hydrochloride, and glutamic hydrochloride), and the like. These compounds may be hydrous or anhydrous, and they can be used alone or in combination of two or more thereof.

Among them, preferred are carboxylic acids such as fumaric acid, adipic acid, malic acid, acetic acid, tartaric acid, fumaric acid, citric acid, and the like, more preferably, tartaric acid, fumaric acid or citric acid which is a solid at ordinary temperature, with citric acid being particularly preferred.

The base used in the present invention may be a solid or a liquid at ordinary temperature (15 to 25°C) but, preferably, a solid basic compound is used. Examples of the "base" include hydroxides (e.g., potassium hydroxide, calcium hydroxide, sodium hydroxide, magnesium hydroxide), carbonates (e.g., sodium carbonate, sodium hydrogen carbonate, potassium carbonate, potassium hydrogen carbonate), amine compounds, amide compounds, ketone compounds, and the like. These compounds may be hydrous or anhydrous, and they can be used alone or in combination of two or more thereof.

In addition to the above ingredients, optionally, the solid preparation of the present invention may contain a medicinal ingredient other than that with tendency toward gelation, and excipient, disintegration agent, binder, lubricant, colorant, flavor, and light-screening agent which are conventionally used in a solid preparation.

The "medicinal ingredient" is not particularly limited and, when the medicinal ingredient with tendency toward gelation is, for example, a compound represented by the formula (I), it can be used in combination with a reverse transcriptase inhibitor and/or protease inhibitor. Specific examples of such drugs include zidovudine, didanosine, zalcitabine, lamivutide, stavudine, saquinavir, ritonavir, indinavir, nelfinavir, and the like.

Examples of the "excipient" include lactose, starch, glucose, mannitol, microcrystalline cellulose, colloidal silicon dioxide, magnesium carbonate, calcium carbonate, calcium phosphate, calcium sulfate, aluminum silicate, aluminum metasilicate, and the like.

Examples of the "disintegration agent" include carboxymethylcellulose calcium, croscarmellose sodium, carboxymethyl starch sodium, starch, low-substituted hydroxypropylcellulose, crosslinked insoluble polyvinylpyrrolidone, and the like.

Examples of the "binder" include hydroxypropylcellulose, α-starch, sucrose, gelatin, Acacia, methylcellulose, hypromellose, carboxymethylcellulose, carboxymethylcellulose sodium, polyvinylpyrrolidone, microcrystalline cellulose, dextrin, pullulan, and the like.

Examples of the "lubricant" include stearic acid, calcium stearate, magnesium stearate, talc, colloidal silica, and the like.

Examples of the "colorant" include yellow ferric oxide, red ferric oxide, and the like.

The "flavor" may be any of synthetic and naturally occurring compounds, and examples thereof include lemon flavor, lime flavor, orange flavor, strawberry flavor, menthol, and the like.

Examples of the "light-screening agent" include titanium dioxide, talc, calcium carbonate, magnesium carbonate, and the like.

One preferred aspect of the present invention is the solid preparation containing talc and/or magnesium stearate in addition to the above ingredients.

The solid preparation of the present invention is in the form of, for example, a circular or oval plain tablet and a coated tablet thereof, with a coated preparation being preferred.

The content of the medicinal ingredient with tendency toward gelation in the solid preparation of the present invention is no particularly limited but, in general, it is 0.1 to 45% (w/w) based on the total solid preparation.

The solid preparation of the present invention contains 0.1 to 20 parts by weight, preferably 1 to 10 parts by weight, and more preferably 1 to 5 parts by weight of the acid or the base relative to 1 part by weight of the medicinal ingredient with tendency toward gelation.

Further, the solid preparation of the present invention contains 0.05 to 20 parts by weight, preferably 0.1 to 15 parts by weight, and more preferably 0.5 to 5 parts by weight of the surface modifier relative to 1 part by weight of the medicinal ingredient with tendency toward gelation.

The content of the acid or the base relative to the total amount of the solid preparation of the present invention (a plain tablet in case of a coated preparation) is preferably 2 to 85% (w/w), more preferably 5 to 65% (w/w), and more preferably 8 to 17% (w/w).

### Production process

The solid preparation of the present invention can be produced by combining applicable known processes according to a particular dosage form. The production conditions of each step can be determined according to a conventional method. However, at this time, it is important to mix the surface modifier with at least one of the medicinal ingredient with tendency toward gelation and the acid or the base prior to mixing the medical ingredient and the acid or the base. Further, preferably, the acid (or the base) is mixed with the surface modifier in advance and, in particular, each of the medicinal ingredient with tendency toward gelation and the acid or base is mixed with the surface modifier in advance. The solid preparation produced in this manner can be expected to have such a structure that the surface modifier adheres to the surface of the medicinal ingredient with tendency toward gelation and/or the acid (or the base) in the form of small clumps, thereby expecting exertion of excellent effects. However, the present invention is not limited to this.

A preferred production process will be described below.

The medicinal ingredient with tendency toward gelation (hereinafter, sometimes, referred to as the "medicinal component") and the surface modifier are mixed in order to modify the surface of the medicinal ingredient. Usually, a mixer such as a tumbler mixer or a device having a stirring mechanism such as a kneader/granulator is used for mixing the ingredients. Similarly, the surface modifier and the acid (or the base) are mixed in order to modify the surface of the acid (or the base). Then, these are mixed with a binder, followed by granulation to prepare granules. The mixing and granulation are carried out using a conventional granulator. At this time, granulation can also be carried out after the binder has been mixed with a mixture (premix) obtained by premixing a excipient and/or a disintegration agent and the medicinal ingredient and/or an acidic compound. The mixing and granulation of the binder and the medicinal ingredient and/or the acid (or the base) are preferably carried out at about 0 to 100°C. The content of the binder in the granules is about 0.1 to 50 wt%. The contents of the excipient and the disintegration agent in the granules are about 1 to 99.9 wt% and about 0.1 to 50 wt%, respectively. The resulting granules contain 50% or more of particles of 50 µm to 1.5 mm (preferably 50% or more of particles of 150 µ m to 1.0 mm). For removing water, the resulting granules may be dried for about 0.01 to 72 hours at about 10 to 80°C. In addition, the granules thus prepared may be sized. Usually, the sizing is carried out using a commercially available classifier such as a power mill. The sized granules preferably contain 50% or more of particles of about 50 µm to 1.5 mm (preferably 50% or more of particles of 150 mm to 1.0 mm). Croscarmellose sodium and another disintegration agent, and magnesium stearate or another lubricant may also be added thereto. Further, the acid (or the base) that has been subjected to surface modification may further be added. Alternatively, the acid (or the base) may be used as such without subjecting to surface modification. For carrying out this mixing, usually, a commercially available mixer such as a tumbler mixer is used. The amounts of the disintegration agent and the lubricant are somewhat larger than those conventionally employed in preparations, and are about 0.1 to 50 wt% and 0.1 to 10 wt%, respectively.

Although the resulting mixed granules may be used as such, usually, the granules are processed in the form of pills, tablets, capsules, or the like. Preferably, they are molded in the form of tablets such as circular, oval or oblong tablets. For molding, a commercially available molding device such as a tableting machine is used. The compression pressure used in molding tables is usually about 1 to 25 kN. Usually, a circular tablet has a diameter of about 5 to 20 mm and a thickness of about 1 to 10 mm. An oval-type tablet usually has a major diameter of 7 to 20 mm and a minor diameter of about 5 to 15 mm, with a thickness of about 1 to 10 mm. An oblong tablet usually has a major diameter of about 7 to 20 mm, a minor diameter of about 5 to 15 mm, and a thickness of about 1 to 10 mm. For producing a coated preparation, the table obtained above may be subjected to a film coating process. The film coating process is usually carried out with a pan coating device or the like. Examples of the film-coated tablet include tablets produced by forming a film coating on circular tablets, oval tablets, or oblong tablets. A film-coating solution can be prepared by dissolving or dispersing hypromellose or another high molecular weight material for film-coating in a solvent such as water. A colorant and a light-screening agent may also be blended in the film coating solution. Preferably, the temperature of the product (tablets) is controlled at about 10 to 100°C when spraying the film coating solution. More preferably, the temperature is controlled at about 30 to 80°C, and furthermore preferably, the temperature is controlled at about 40 to 60°C.

That is, the solid preparation of the present invention can be produced, for example, by a production process comprising the following steps of step A and/or step A', and step B.
Step A: Mixing an acid (or a base) with a surface modifier; Step A': Optionally, mixing a surface modifier and a medicinal ingredient with tendency toward gelation;
Step B: After step A (and/or step A'), mixing a mixture of the acid (or the base) and the surface modifier with the medicinal ingredient with tendency toward gelation (or a mixture of the surface modifier and the medicinal ingredient with tendency toward gelation).

Herein, it is desirable that the acid (or the base) and the medicinal ingredient with tendency toward gelation is uniformly mixed. For this purpose, each of the acid (or the base) and the medicinal ingredient with tendency toward gelation is preferably in the form of a powder. In particular, it is preferable that 70 wt% or more (e.g., 70 to 90 wt%, preferably 80 wt% or more) of each powder can pass through a 75 µm mesh. Usually, an average particle diameter of such a powder is about 1 µm or more. When the acid (or the base) is a liquid, it can be used by allowing it to be absorbed in a carrier with a similar particle size (e.g., colloidal silicon dioxide (Aerosil; manufactured by Japan Aerosil), calcium silicate, or aluminum silicate).

The surface modifier preferably has a particle size of 1/5-fold or less, preferably 1/10-fold or less, more preferably 1/100-fold or less as large as the particle size of the powder of the medicinal ingredient with tendency toward gelation or the acid (or the base). Specifically, the preferred average particle size is about 5 nm to 5 µm, more preferably about 7 nm to 100 nm, in particular, about 10 nm to 50 nm.

The term "average particle diameter" used herein refers to a median diameter based on the number of particles.

After the above steps, in cases that the solid preparation of the present invention is a tablet, a mixture containing the medicinal ingredient with tendency toward gelation, the surface modifier, and the acid (or the base) is compressed according to a conventional method to obtain a tablet. Further, optionally, the tablet thus obtained is subjected to coating according to a conventional method to obtain the coated preparation.

Ingredients other than the aforementioned medicinal ingredient with tendency toward gelation, surface modifier, and acid (or base) can be added in appropriate steps as with a conventional production process of a solid preparation.

The solid preparation of the present invention can be administered to a patient following a conventional administration method.

The following Examples, test examples, etc. will explain the present invention in more detail, but the present invention is not restricted thereto.

### Example 1

(S)-8-[4-(2-Butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide monomethanesulfonate (hereinafter referred to as "compound A") (56.9 g), mannitol (433.1 g), citric acid anhydride (100 g), colloidal silicon dioxide (Aerosil; 16 g), and microcrystalline cellulose (110 g) were uniformly mixed in a fluidized-bed granulator-dryer. Then, granulation was carried out with spraying an aqueous solution prepared by dissolving hydroxypropylcellulose (HPC-L; 16 g) in water in the device, followed by drying in the same device. The resulting granules were screened and sized with a 16-mesh screen. Further, 732 g of the granules were taken out, croscarmellose sodium (Ac-Di-Sol; 40 g), talc (16 g), and magnesium stearate (12 g) were added thereto and the mixture was mixed with a tumbler mixer to obtain mixed granules. The mixed granules were then compressed into tablets each weighing 400 mg with 9.5 mm diameter punch in a tableting machine to prepare plain tablets.

### Example 2

Compound A (56.9 g), citric acid anhydride (150 g), and colloidal silicon dioxide (Aerosil; 24 g) were mixed thoroughly in a bag in advance, and compound A and colloidal silicon dioxide were subjected to surface modification. Then, the mixture of the base drug/acid-surface modifier (230.9 g), mannitol (683.1 g), and microcrystalline cellulose (160 g) were mixed uniformly in a fluidized bed granulator-dryer, and granulation was carried out by spraying an aqueous solution prepared by dissolving hydroxypropylcellulose (HPC-L; 24 g) in water in the device, followed by drying in the same device. The resulting granules were screened and sized with a 16-mesh screen. Further, 1098 g of the granules were taken out, croscarmellose sodium (Ac-Di-Sol; 60 g), talc (24 g), and magnesium stearate (18 g) were added thereto, and the mixture was mixed with a tumbler mixed to obtain mixed granules. The mixed granules were then compressed in tablets each weighing 600 mg with 13.5 x 8.5 mm (diameter) oblong punch in a tableting machine to prepare plain tablets.

### Reference Example 1

Compound A (120 g), mannitol (171.6 g), and microcrystalline cellulose (36 g) were mixed thoroughly in a fluidized bed granulator-dryer, and granulation was carried out by spraying an aqueous solution prepared by dissolving hydroxypropylcellulose (HPC-L; 10.8 g) in water in the device, followed by drying in the same device. The resulting granules were screened and sized with a 16-mesh screen. Further, 282 g of the granules were taken out, croscarmellose sodium (Ac-Di-Sol; 15 g) and magnesium stearate (3 g) were added thereto, and the mixture was mixed in a bag to prepare mixed granules. The mixed granules were then compressed into tablets each weighing 300 mg with 9.5-mm diameter punch in a tableting machine to obtain plain tablets. A film coating solution composed of hypromellose (19.6 g), polyethylene glycol 6000 (4 g), titanium dioxide (2 g), and yellow ferric oxide (0.4 g) was sprayed on the above obtained tablets with a pan coater (Hicoater, Freund) to obtain film-coated tablets. At this time, conditions were controlled so that the product temperature was 30 to 50°C.

### Reference Example 2

Compound A (56.9 g), mannitol (449.1 g), citric acid anhydride (100 g), and microcrystalline cellulose (110 g) were uniformly mixed, and granulation was carried out by spraying an aqueous solution prepared by dissolving hydroxypropylcellulose (HPC-L; 16 g) in water in a device, followed by drying in the same device. The resulting granules were screened and sized with a 16-mesh screen. Further, 732 g of the granules were taken out, croscarmellose sodium (Ac-Di-Sol; 40 g), talc (16 g), and magnesium stearate (12 g) were added thereto and the mixture was with a tumbler mixer to obtain mixed granules. The mixed granules were then compressed into tablets each weighing 400 mg with 9.5-mm diameter punch in a tableting machine to obtain plain tablets.

### Test Example 1

The tables of Reference Example 1 do not contain a surface modifier and an acid, or a base. The tablets of Reference Example 2 do not contain a surface modifier but contain citric acid anhydride. The tablets of Example 1 contain colloidal silicon dioxide as a surface modifier and citric acid anhydride. The tablets of Example 2 contain colloidal silicon dioxide as a surface modifier and citric acid anhydride, and compound A and citric acid anhydride are surface-treated with colloidal silicon dioxide. During the production of tablets of Reference Examples 1 and 2, poor fluidity in the fluidized bed granulator was observed and inferior workability was experienced.

These tablets were subjected to disintegration testing, dissolution testing, and stability testing.

The disintegration testing was carried out according to the Japanese Pharmacopoeia disintegration test method.

The results of the disintegration testing are shown in Table 1. As seen from the table, the preparation of Reference Example 1 was not completely disintegrated. Although the preparation of Reference Example 2 was disintegrated, it was required 30 min or longer period of time. On the other hand, the preparation of Example 1 had superior disintegration properties, and the preparation of Example 2 had further superior disintegration properties.

The dissolution testing was carried out according to the Japanese Pharmacopoeia paddle method under the conditions of 50 rpm, 37°C, and n=6. As a test solution, 900 mL of the first disintegration solution (pH 1.2) containing 0.1% cetyltrimethylammonium bromide was used. The results of the dissolution testing are shown in Fig. 1. As seen from the figure, dissolution of the preparation of Reference Example 2 was slow, while the dissolution of the active ingredient from the preparation of Example 1 was excellent, and that of the preparation of Example 2 was more excellent.

As shown in Table 2, product quality (properties, residual ratio of the active ingredient) was tested after storage for 3 months under the conditions of 40°C in a sealed glass vial. As seen from this table, while a decrease in the residual ratio and a change in properties were observed in Reference Example 2, the preparations of Examples 1 and 2 had good stability because the decrease in the residual ratio was improved and no significant change of properties was found.

In these testing, the measurement of compound A and related substances were carried out by high performance liquid chromatography under the following measurement conditions.

### Measurement conditions

Detector: Ultraviolet spectrophotometer (detection wavelength: 242 nm)
Column: YMC-Pack Pro C18, 3 mm, 4.6 mm i.d. x 100 mm (YMC Co.)
Column temperature: Constant temperature about 20°C
Mobile phase: Acetonitrile/0.05 mol/L ammonium acetate solution/methanol solution (12:7:1)
Flow rate: About 1 mL/min

**Table 1**

| | Reference Example 1 | Reference Example 2 | Example 1 | Example 2 |
|---|---|---|---|---|
| Disintegration time | No disintegration | > 30 min. | 23 min. | 6 min. |

**Table 2**

| | Residual ratio (%) |
|---|---|
| Reference Example 2 (initial) | (100) |
| Reference Example 2, 40°C, 3M | 96.9 |
| Example 1 (initial) | (100) |
| Example 1, 40°C, 3M | 98.6 |
| Example 2 (initial) | (100) |
| Example 2, 40°C, 3M | 101.4 |

### Example 3

A preparation was obtained according to the formulation as shown in Table 3. For 25-mg tablets, compound A (247.9 g), citric acid anhydride (645 g), and colloidal silicon dioxide (Aerosil; 103.2 g) were first thoroughly mixed in a bag to modify the surface of compound A and citric acid anhydride. Then, the bases drug/acid-surface modifier mixture (996.1 g), mannitol (2934 g), and microcrystalline cellulose (688 g) were uniformly mixed in a fluidized bed granulator-dryer, and granulation was carried out by spraying an aqueous solution prepared by dissolving hydroxypropylcellulose (HPC-L; 103.2 g) in water in the device, followed by drying in the same device. The resulting granules were then milled and sized with a powder mill and a 1.5 mm diameter punching screen. Further, 4008 g of the granules were taken out, croscarmellose sodium (Ac-Di-Sol; 219 g), talc (87.6 g), and magnesium stearate (65.7 g) were added thereto, and the mixture was mixed with a tumbler mixer to obtain mixed granules. The mixed granules were then compressed into tablets each weighing 600 mg with a 13.5 mm x 8.5 mm (diameter) oblong punch in a tableting machine to obtain plain tablets. A film coating solution composed of hypromellose (139 g), polyethylene glycol 6000 (32 g), titanium dioxide (16 g), and yellow ferric oxide (3 g) was sprayed with a pan coater (Driacoater, Powrex Corp.) to obtain film-coated tablets. At this time, the conditions were controlled so that the product temperature was 40 to 50°C. Similarly, 5 mg tablets were prepared by adjusting the content of compound A and mannitol.

**Table 3**

| | 5 mg | 25 mg |
|---|---|---|
| | 5.69 mg | 28.45 mg |
| Compound A | (5 mg as free form) | (25 mg as free form) |
| Citric acid anhydride | 75 mg | 75 mg |
| Colloidal silicon dioxide | 12 mg | 12 mg |

| (Aerosil) | | |
|---|---|---|
| Mannitol | 364.31 mg | 341.55 mg |
| Microcrystalline cellulose. | 80 mg | 80 mg |
| Hydroxypropylcellulose | 12 mg | 12 mg |
| Croscarmellose sodium | 30 mg | 30 mg |
| Magnesium stearate | 9 mg | 9 mg |
| Talc | 12 mg | 12 mg |
| Plain tablet total | 600 mg | 600 mg |
| Hypromellose | 13.9 mg | 13.9 mg |
| Polyethylene glycol 6000 | 3.2 mg | 3.2 mg |
| Titanium dioxide | 1.6 mg | 1.6 mg |
| Yellow ferric oxide | 0.3 mg | 0.3 mg |
| Tablet total | 619 mg | 619 mg |

### Industrial Applicability

According to the present invention, it is possible to improve the disintegration properties of a solid preparation comprising a medicinal ingredient with tendency of gelation.

According to the present invention, it is possible to improve the workability of the above solid preparation having improved disintegration properties.

According to the present invention, it is possible to improve the stability of the above solid preparation having improved disintegration properties.

## Claims

1. A solid preparation comprising a medicinal ingredient having tendency toward gelation, a surface modifier, and an acid or base.

2. The solid preparation according to claim 1, wherein the medicinal ingredient having tendency toward gelation is a salt of a compound that is extremely poorly soluble in the state of its free form.

3. The solid preparation according to claim 1, wherein the medicinal ingredient having tendency toward gelation is a salt of an amphoteric or basic compound, and the acid or base is an acid.

4. The solid preparation according to claim 2, wherein the salt is that formed with hydrochloric acid, hydrobromic acid, nitric acid, sulfuric acid, phosphoric acid, formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, arginine, lysine, or ornithine.

5. The solid preparation according to claim 1, wherein the amphoteric or basic compound, or salt thereof, is a compound represented by the formula (I): 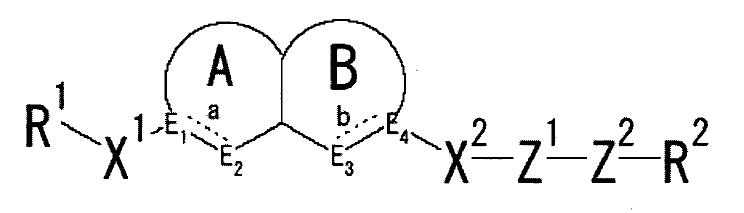 wherein R¹ denotes an optionally substituted 5- to 6-membered ring,
X¹ denotes a bond or a divalent group wherein the number of atoms constituting the straight-chain moiety is 1 to 4, ring A denotes an optionally substituted 5- or 6-membered ring and ring B denotes an optionally substituted 8- to 10-membered ring,
E₁ and E₄ each denote an optionally substituted carbon atom or an optionally substituted nitrogen atom,
E₂ and E₃ each denote an optionally substituted carbon atom, an optionally substituted nitrogen atom, an optionally oxidized sulfur atom or an oxygen atom,
a and b each denote a single bond or a double bond,
X² denotes a divalent group wherein the number of atoms constituting the straight-chain moiety is 1 to 4,
Z¹ denotes a bond or a divalent cyclic group,
Z² denotes a bond or a divalent group,
R² denotes (1) an optionally substituted amino group wherein the nitrogen atom may be converted into a quaternary ammonium or oxide, (2) an optionally substituted nitrogen-containing heterocyclic group which may contain a sulfur atom or an oxygen atom as a ring-constituent atom and wherein the nitrogen atom may be converted into a quaternary ammonium or oxide, (3) a group represented by the formula: 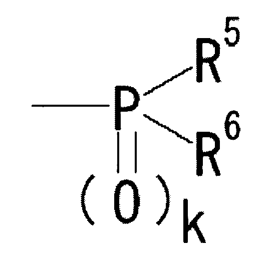 wherein k denotes 0 or 1, and when k is 0, the phosphorus atom can form a phosphonium salt, R⁵ and R⁶ each denote an optionally substituted hydrocarbon group, an optionally substituted hydroxyl group, or an optionally substituted amino group, or R⁵ and R⁶ can bond each other to form a cyclic group together with the adjacent phosphorus atom, (4) an optionally substituted amidino group, or (5) an optionally substituted guanidino group, or a salt thereof.

6. The solid preparation according to claim 1, wherein the acid or base is a solid.

7. The solid preparation according to claim 1, wherein the acid is a carboxylic acid, sulfonic acid, an acidic polysaccharide, or an acidic amino acid.

8. The solid preparation according to claim 1, wherein the acid is a carboxylic acid.

9. The solid preparation according to claim 8, wherein the carboxylic acid is fumaric acid, adipic acid, malic acid, acetic acid, tartaric acid, succinic acid or citric acid.

10. The solid preparation according to claim 9, wherein the carboxylic acid is citric acid.

11. The solid preparation according to claim 1, which comprises 0.1 to 20 parts by weight of the acid or base based on 1 part by weight of the medicinal ingredient with tendency toward gelation.

12. The solid preparation according to claim 1, which comprises 0.05 to 20 parts by weight of the surface modifier based on 1 part by weight of the medicinal ingredient with tendency toward gelation.

13. The solid preparation according to claim 1, which is a tablet.

14. The solid preparation according to claim 1, which is a coated preparation

15. The solid preparation according to claim 1, wherein the content of the acid or base is 2 to 85% (w/w) based on the total preparation.

16. The solid preparation according to claim 1, wherein the content of the acid or base is 2 to 85% (w/w) based on a plain tablet.

17. The solid preparation according to claim 1, which further comprises talc and/or magnesium stearate.

18. A process for producing a solid preparation which comprises a medicinal ingredient with tendency toward gelation a surface modifier, and an acid or base, wherein at least the acid or base is mixed in advance with colloidal silicon dioxide.

19. The process according to claim 18, wherein the average particle diameter of the surface modifier is as large as 1/5 or less of that of the medicinal ingredient with tendency toward gelation in the form of a powder.

20. The process according to claim 18, wherein the average particle diameter of the surface modifier is as large as 1/5 or less of that of the acid or base in the form of a powder.

21. The process according to claim 18, wherein the average particle diameter of the surface modifier is about 5 nm to 5 µm.
